(19) Europäisches Patentamt
European Patent Office
Office européen des brevets

(11) **EP 4 455 674 A1**

(12) **EUROPEAN PATENT APPLICATION**
published in accordance with Art. 153(4) EPC

(43) Date of publication:
**30.10.2024 Bulletin 2024/44**

(21) Application number: **22910873.3**

(22) Date of filing: **06.12.2022**

(51) International Patent Classification (IPC):
*G01N 35/00* (2006.01)   *B04B 5/04* (2006.01)
*B04B 9/10* (2006.01)   *B04B 13/00* (2006.01)
*G01N 1/10* (2006.01)   *G01N 33/48* (2006.01)
*G01N 37/00* (2006.01)

(52) Cooperative Patent Classification (CPC):
**B04B 5/04; B04B 9/10; B04B 13/00; G01N 1/10;
G01N 33/48; G01N 35/00; G01N 37/00**

(86) International application number:
**PCT/JP2022/044876**

(87) International publication number:
**WO 2023/120159 (29.06.2023 Gazette 2023/26)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **20.12.2021 JP 2021206453
13.01.2022 JP 2022003496**

(71) Applicant: **PHC Holdings Corporation
Tokyo 100-8403 (JP)**

(72) Inventors:
• **MATSUBARA, Fumiya
Toon-shi, Ehime 791-0395 (JP)**
• **NISHIO, Akira
Toon-shi, Ehime 791-0395 (JP)**

(74) Representative: **Novagraaf International SA
Chemin de l'Echo 3
1213 Onex, Geneva (CH)**

(54) **BIOLOGICAL SAMPLE SEPARATION CONTAINER, BIOLOGICAL SAMPLE SEPARATION
CONTROL DEVICE, BIOLOGICAL SAMPLE SEPARATION CONTROL METHOD, AND
BIOLOGICAL SAMPLE SEPARATION CONTROL PROGRAM**

(57)    A container (430) includes a isolation chamber (431), a collection chamber (432), a waste liquid holding chamber (433), a hydrophilic first flow path (434), and a hydrophilic second flow path (435). In the first flow path (434), when capillary action is exerted and centrifugal force is applied, the specific component is moved from the isolation chamber (431) to the collection chamber (432) by the siphon principle. In the second flow path (435), when a capillary action with a different magnitude from that of the first flow path (434) is exerted and centrifugal force is applied, the waste liquid is moved from the isolation chamber (431) to the waste liquid holding chamber (433) according to the siphon principle. The first flow path (434) is connected at a first position on a first side of the isolation chamber (431), and the second flow path (435) is connected at a second position on a second side that is on the opposite side from the first side in the isolation chamber (431), and the first position and the second position are offset in the radial direction from each other.

FIG. 12

**Description**

TECHNICAL FIELD

The present invention relates to a biological sample isolation container and a biological sample isolation control device that are used in a state of being placed in a rotation device and subjected to centrifugal force.

BACKGROUND ART

Blood or another such biological sample has been put in a centrifuge tube or another such container and subjected to centrifuging in the past in order to isolate a specific component contained in the blood.

The work of extracting and collecting a specific component of a biological sample isolated into components by centrifugation from a container is sometimes performed manually by an operator.

For example, Patent Literature 1 discloses a sample preparation device comprising a dispensing unit having a nozzle that aspirates reagent from a reagent container and discharges it into a reaction container, a shutter member that opens and closes an insertion opening formed in a lid section that covers the top of a reagent installation unit in which a reagent container is installed, in order to improve the airtightness of the reagent installation unit while allowing the reagent to be aspirated with a nozzle for sample preparation.

CITATION LIST

PATENT LITERATURE

Patent Literature 1: JP-A 2021-162325

(TECHNICAL PROBLEM)

However, the following problem was encountered with the conventional sample preparation apparatus described above.

With the sample preparation device disclosed in the above-mentioned publication, the reagent aspirated from the reagent container is subjected to treatment with a dispensing unit that dispenses the reagent into a reaction container containing a specimen, and the specimen and reagent are allowed to react in the reaction container, after which centrifugation is performed. After centrifugation has been performed and the sample and reagent are stirred, the supernatant in the reaction container is aspirated through a nozzle.

Here, the accuracy of collecting the supernatant depends on the accuracy of nozzle operation, and variance may occur.

It is an object of the present invention to provide a biological sample isolation container, a biological sample isolation control device, a biological sample isolation control method, and a biological sample isolation control program with which it is less likely that variance in collection accuracy will occur when extracting and collecting a specific component of a biological sample isolated into components from a container.

(SOLUTION TO PROBLEM)

The biological sample isolation container according to the present invention is a biological sample isolation container for recovering a specific component contained in a biological sample isolated into components, by rotating in a state of being placed in a rotation device, comprising a isolation unit, a collection unit, a waste liquid holding unit, a hydrophilic first flow path, and a hydrophilic second flow path. The isolation unit holds a biological sample and a reagent. The collection unit is disposed more to the outside in the radial direction than the center of rotation with respect to the isolation unit in a state of being placed in the rotation device, and collects a specific component of a biological sample. The waste liquid holding unit is disposed more to the outside in the radial direction than the center of rotation with respect to the isolation unit in a state of being placed in the rotation device, and holds waste liquid of the biological sample and the reagent, excluding the specific component. The hydrophilic first flow path connects the isolation unit and the collection unit, and when capillary action is exerted and centrifugal force is applied due to rotation, the specific component is moved from the isolation unit to the collection unit by the siphon principle. The hydrophilic second flow path connects the isolation unit and the waste liquid holding unit, and when capillary action having a different magnitude from that of the first flow path is exerted and centrifugal force is applied due to rotation, the waste liquid is moved from the isolation unit to the waste liquid holding unit by the siphon principle. The first flow path is connected at a first position on a first side of the isolation unit. The second flow path is connected at a second position on a second side of the isolation unit on the opposite side from the first side. The first position and the second position are offset in the radial direction from each other in a state of being placed in the rotation device.

(EFFECTS)

With the biological sample isolation container according to the present invention, it is less likely that variance in collection accuracy will occur when extracting and collecting a specific component of a biological sample isolated into components from a container.

BRIEF DESCRIPTION OF THE DRAWINGS

Fig. 1 is an overall oblique view of the configuration of a biological sample isolation device in which the biological sample isolation control device according to an embodiment of the present invention is installed;
Fig. 2 is an oblique view of the configuration of the biological sample isolation device in Fig. 1 when the pullout section is open;
Fig. 3 is an exploded oblique view of the configuration of the biological sample isolation device in Fig. 1;
Fig. 4 is a control block diagram of the biological sample isolation device in Fig. 1;
Fig. 5 is an oblique view of the configuration of a container that is rotated by the biological sample isolation device in Fig. 1 to stack blood and a specific gravity adjusting agent;
Fig. 6 is a conceptual diagram showing the centrifugal force that is applied to the blood held in a first holding part of the container and to the specific gravity adjusting agent held in a second holding part when the container in Fig. 5 placed in the container holder of the biological sample isolation device in Fig. 1 is rotated;
Figs. 7A and 7B are conceptual diagrams illustrating the principle by which blood is pumped from a first holding unit to a second holding unit during rotation in the hydrophobic flow path provided to the container in Fig. 5;
Fig. 8 is a graph of the relation between rotation speed and elapsed time, from the processing to stack the blood and the specific gravity adjusting agent by rotating the container in Fig. 5 up to the processing to isolate the blood components, using the biological sample isolation device in Fig. 1;
Fig. 9 is a flowchart of the flow of processing in the biological sample isolation control method shown in Figs. 7A and 7B;
Figs. 10A, 10B, and 10C are plan views of the configuration of a container in which a biological sample and a reagent are stacked by a biological sample isolation control device in another embodiment of the present invention;
Fig. 11 is an oblique view of the configuration of a container that is rotated by the biological sample isolation device in Fig. 1 to isolate and collect a specific component from a state in which blood and a specific gravity adjusting agent are stacked in Embodiment 2 of the present invention;
Fig. 12 is a conceptual diagram showing the centrifugal force exerted on the blood and specific gravity adjusting agent held in a state of being stacked in the isolation chamber of the container in the rotation of the container in Fig. 11 placed in the container holder of the biological sample isolation device in Fig. 1;
Figs. 13A and 13B are conceptual diagrams illustrating the principle of isolating and collecting a specific blood component during rotation in hydrophilic first and second flow paths provided to the container in Fig. 12;
Figs. 14A, 14B, and 14C are plan views showing the steps of isolating and collecting the specific blood component from a state in which the blood and the specific gravity adjusting agent are stacked, by changing the rotation speed of the rotating container shown in Fig. 12 and thereby changing the magnitude of the centrifugal force on the blood, etc.;
Figs. 15A, 15B, and 15C are plan views showing the steps following Fig. 14C;
Fig. 16 is a graph of the relation between rotation speed and elapsed time in the step of performing processing to isolate and recover the specific component from a state in which the blood and the specific gravity adjusting agent have been stacked by rotation of the container in Fig. 11 using the biological sample isolation device in Fig. 1;
Fig. 17 is a flowchart showing the flow of processing in the biological sample isolation control method shown in Fig. 16;
Fig. 18 is a graph of the relation between rotation speed and elapsed time in the step of performing processing to isolate and recover the specific component from a state in which the blood and the specific gravity adjusting agent have been stacked in the biological sample isolation control method according to yet another embodiment of the present invention;
Figs. 19A and 19B are oblique views of the configuration in Embodiment 3 of the present invention in which the blood and the specific gravity adjusting agent have been stacked in the biological sample isolation control method of Fig. 1, and the configuration of the container in which the specific component is isolated and collected after stacking;
Figs. 20A and 20B are conceptual diagrams showing the centrifugal force applied to the blood held in a first holding part of the container and the specific gravity adjusting agent held in a second holding part when the container shown in Fig. 19A, etc., has been placed in the container holder of the biological sample isolation device in Fig. 1;

Figs. 21A, 21B, and 21C are plan views of the steps showing the steps of isolating and collecting the specific blood component from a state in which the blood and the specific gravity adjusting agent are stacked, by changing the rotation speed of the rotating container shown in Fig. 19A, etc., and thereby changing the magnitude of the centrifugal force on the blood, etc.;

Figs. 22A, 22B, 22C, and 22D are plan views showing the steps following Fig. 21C; and

Fig. 23 is a graph of the relation between rotation speed and elapsed time in the step of performing processing to isolate and recover the specific component from a state in which the blood and the specific gravity adjusting agent have been stacked by rotation of the container in Fig. 19A, etc., using the biological sample isolation device in Fig. 1.

DESCRIPTION OF THE EMBODIMENTS

Embodiment 1

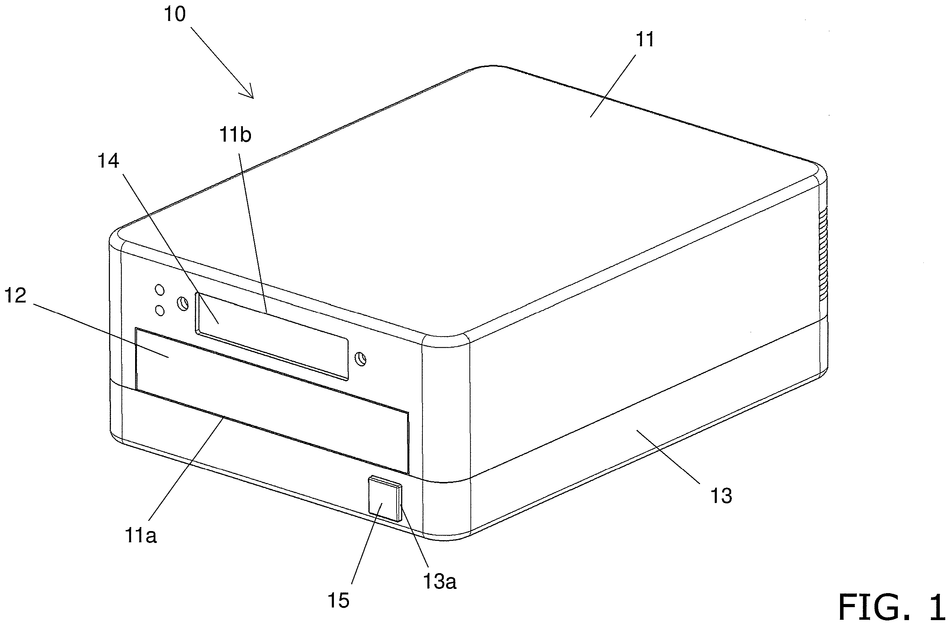

A biological sample isolation device (rotation device) 10 equipped with a biological sample isolation control device (control unit 20), and a biological sample isolation control method, according to an embodiment of the present invention will now be described through reference to Figs. 1-9.

In this embodiment, some unnecessarily detailed description may be omitted. For example, detailed description of already known facts or redundant description of components that are substantially the same may be omitted. This is to avoid unnecessary repetition in the following description, and facilitate an understanding on the part of a person skilled in the art. The applicant has provided the appended drawings and the following description so that a person skilled in the art might fully understand this disclosure, but does not intend for these to limit what is discussed in the patent claims.

(1) Configuration of Biological Sample Isolation Device 10

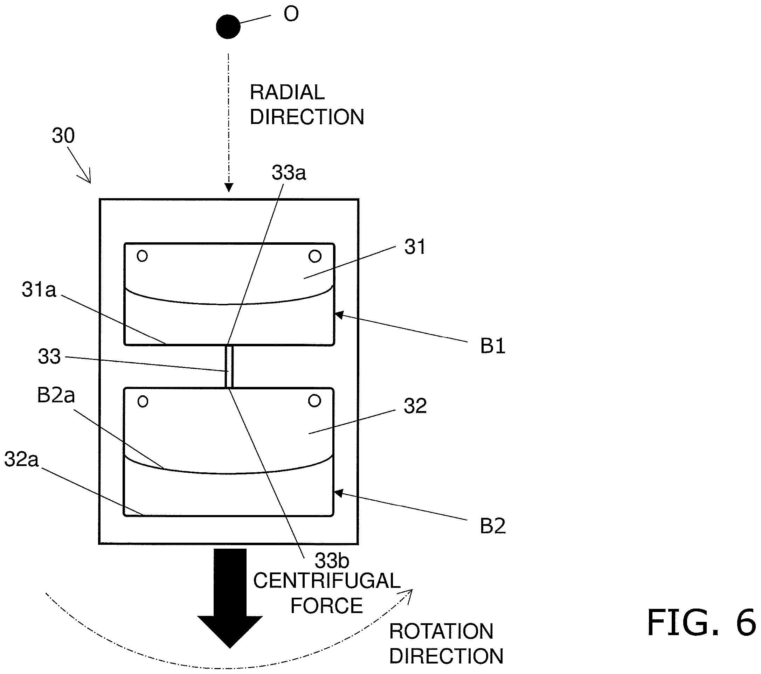

The biological sample isolation device (rotation device) 10 according to this embodiment is a centrifuge used to stack blood B1 (see Fig. 6) so as not to mix it with the specific gravity adjusting agent B2 in a container 30.

Here, for example, in the step of isolating mononuclear cells from blood (whole blood), the specific gravity adjusting agent is put in a centrifuge vessel, and it is necessary to carry out the work carefully so that the blood and the specific gravity adjusting agent do not mix and instead form a stacked state. If this process of forming a stacked state of blood and a specific gravity adjusting agent is performed manually, there is the risk that the layers after blood cell isolation may not be properly formed.

In view of this, in this embodiment, the control unit 20 controls the rotation of the container 30 by the biological sample isolation device 10 so that the blood and the specific gravity adjusting agent can be appropriately stacked in the container 30.

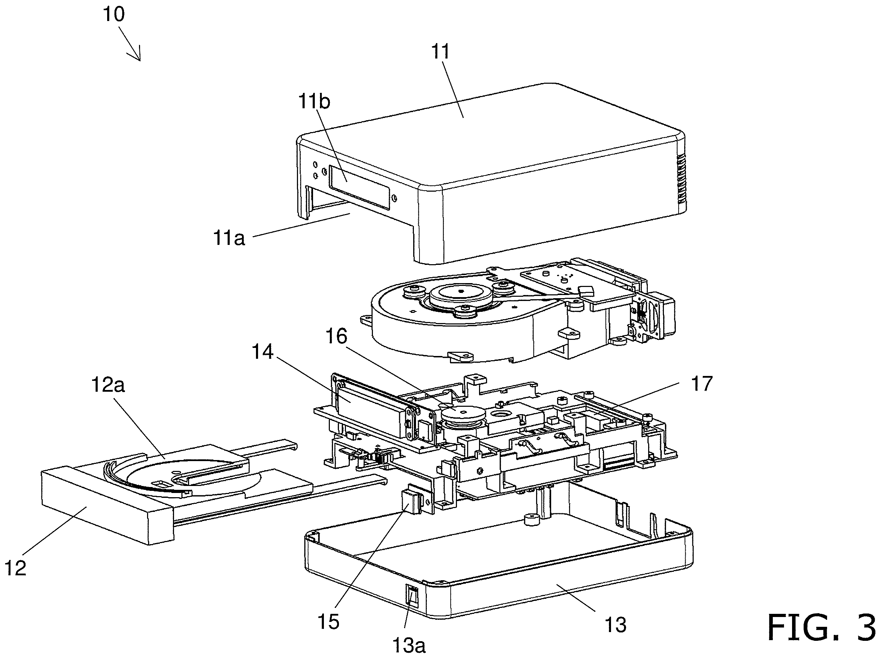

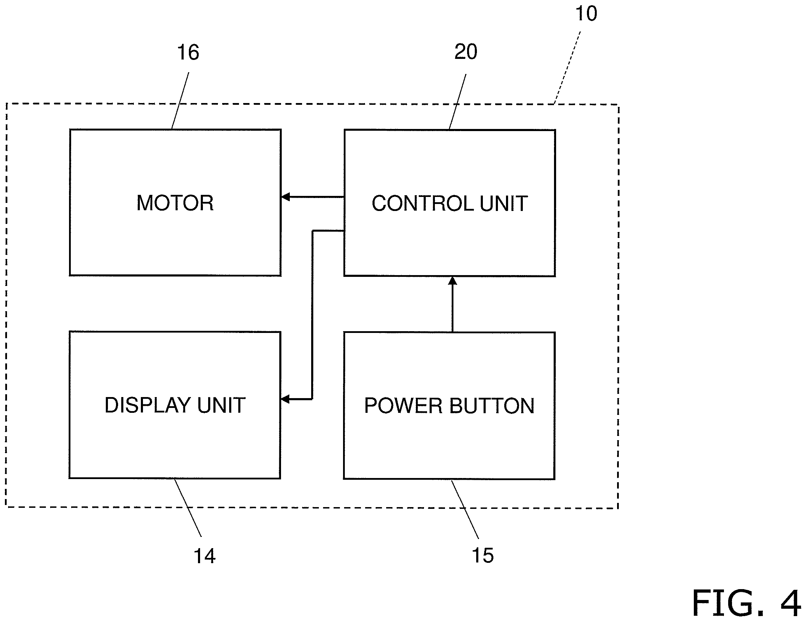

As shown in Fig. 1, the biological sample isolation device 10 comprises a housing unit 11, a pullout section 12, a base member 13, a display unit 14, a power button 15, a motor (rotational drive unit) 16 (see Fig. 3), a board unit 17 (see Fig. 3), and a control unit (biological sample isolation control device, control unit) 20 (see Fig. 4).

As shown in Fig. 1, the housing unit 11 is a box-shaped member that forms the upper contour of the biological sample isolation device 10, the front side of which is provided with an opening 11a through which the pullout section 12 is inserted and removed, and an opening 11b through which the display unit 14 is exposed.

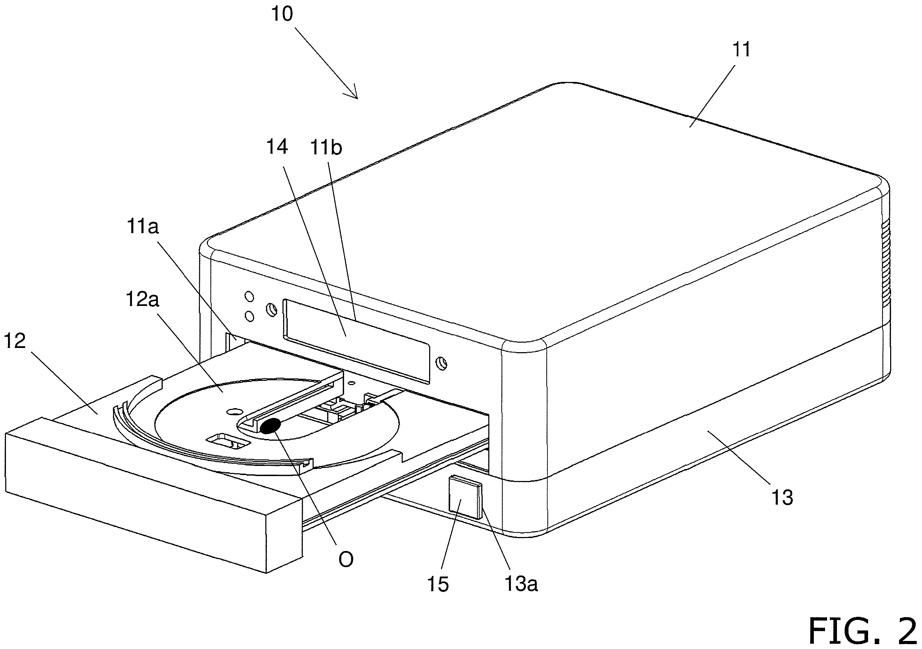

As shown in Fig. 1, the pullout section 12 is attached such that it can be held inside of the housing unit 11, and as shown in Fig. 2, is moved to a state of projecting outside the housing unit 11 by the drive force of the motor (not shown).

As shown in Fig. 2, the container 30 is placed in the container holder 12a provided on the upper surface of the pullout section 12, in a state in which the pullout section 12 has been pulled out to the outside of the housing unit 11.

The containers 30 are placed in container holder 12a such that a plurality of the containers 30 are disposed radially around the rotational center O (see Fig. 2, etc.), for example. Consequently, when the containers 30 are rotated, a centrifugal force facing outward in the radial direction is applied to the blood B1, etc., held in the container 30.

As shown in Figs. 1 and 2, the base member 13 is a box-shaped member that is combined with the housing unit 11 to constitute the outer contour of the biological sample isolation device 10, and is provided to the bottom part of the housing unit 11. As shown in Fig. 3, an opening 13a that exposes the power button 15 (discussed below) is provided on the front surface of the base member 13.

As shown in Figs. 1 and 2, the display unit 14 is provided so as to be exposed on the front side of the housing unit 11, and when the power button 15 is pressed to turn on the power to the biological sample isolation device 10, various kinds of information are displayed. Also, as shown in Fig. 3, the display unit 14 is provided at the end of the board unit 17, and is controlled by a control unit 20 (see Fig. 4) included in the board unit 17.

As shown in Fig. 1, the power button 15 is provided so as to be exposed on the front side of the base member 13, and is pressed to turn the power of the biological sample isolation device 10 on and off. As shown in Fig. 3, the power button 15 is similar to the display unit 14 in that it is provided at the end of the board unit 17, and the power on/off operation is inputted to the control unit 20 included in the board unit 17 (See Fig. 4).

As shown in Fig. 3, the motor 16 is provided inside the housing unit 11 and rotates the container 30. The motor 16 rotates the containers 30 placed in the container holder 12a of the pullout section 12 in a specific rotational direction at a specific rotation speed, and applies centrifugal force to the blood B1, etc., held in the containers 30.

A biological sample isolation control method featuring rotation control of the container 30 by the motor 16 will be described in detail below.

As shown in Fig. 3, the board unit 17 is attached to the upper surface of the base member 13, and is attached so that the pullout section 12 can move forward and backward. The board unit 17 is electrically connected to the display unit 14 and the power button 15 (discussed above), and includes a control board for controlling these.

The control unit (biological sample isolation control device, control unit) 20 includes a CPU and a motor driver IC provided on an electric board attached to the lower surface side of the board unit 17 shown in Fig. 3. As shown in Fig. 4, the control unit 20 is connected to the display unit 14, the power button 15, and the motor 16. The control unit 20 switches the power state of the device through pressing of the power button 15. Also, the control unit 20 controls the display of the display unit 14 and as well as the rotation of the motor 16 .

(2) Configuration of Container 30

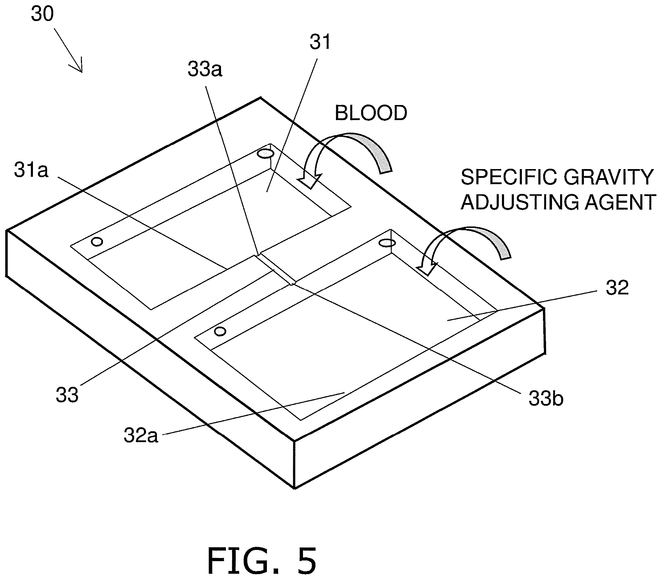

The container 30 used to isolate the components of the blood B1 in the biological sample isolation device 10 of this embodiment will now be described with reference to figs. 5 to 7.

As shown in Fig. 5, the container 30 is sealed in a state in which the blood B1 and the specific gravity adjusting agent B2 are contained therein, and comprises a first holding unit 31 for holding the blood (biological sample) B 1, a second holding unit 32 for holding the specific gravity adjusting agent B2, and a hydrophobic flow path 33 that connects the first holding unit 31 and the second holding unit 32.

As shown in Fig. 6, the first holding unit 31 is disposed radially inward in a state in which the containers 30 have been placed in the container holder 12a of the biological sample isolation device 10, and holds the blood B1.

As shown in Fig. 6, the second holding unit 32 is disposed more radially outward than the first holding unit 31 in a state in which the containers 30 have been placed in the container holder 12a of the biological sample isolation device 10, and hold the specific gravity adjusting agent B2 on which blood B1 is stacked.

As shown in Fig. 6, the first holding unit 31 and the second holding unit 32 are disposed along the radial direction in a state in which the containers 30 have been placed in the container holder 12a of the biological sample isolation device 10.

As shown in Fig. 5, the hydrophobic flow path 33 is a groove formed in the upper surface of the container 30, and is formed as a flow path that produces capillary action. The flow path 33 has a first end 33a that is connected to the inner wall 31a on the outside in the radial direction of the first holding unit 31 and communicates with the space of the first holding unit 31, and a second end 33b that is connected to the inner wall on the inside in the radial direction of the second holding unit 32 and communicates with the space of the second holding unit 32. Also, as shown in Fig. 6, the flow path 33 is disposed along the radial direction in a state in which the containers 30 have been placed in the container holder 12a of the biological sample isolation device 10.

A situation in which the motor 16 of the biological sample isolation device 10 is rotated in a state in which the blood B1 is held in the first holding unit 31 and the specific gravity adjusting agent B2 is held in the second holding unit 32 will now be described through reference to Fig. 6.

At this point, the blood B1 held in the first holding unit 31 of the container 30 and the specific gravity adjusting agent B2 held in the second holding unit 32 are each subjected to centrifugal force outward in the radial direction as shown in Fig. 6.

Consequently, within the first holding unit 31, the blood B1 moves radially outward and is kept in a state of being gathered on the inner wall 31a on the outside in the radial direction of the first holding unit 31. Similarly, within the second holding unit 32, the specific gravity adjusting agent B2 moves radially outward and is kept in a state of being gathered on the inner wall 32a side on the outside in the radial direction of the second holding unit 32.

Consequently, the blood B1 is kept in a state of having moved to the side of the inner wall 31a to which the first end 33a of the flow path 33 is connected. Also, the specific gravity adjusting agent B2 is kept in a state of being gathered on the side of the inner wall 32a that is on the opposite side from the radially inner surface to which the flow path 33 is connected and is spaced apart from the second end 33b of the flow path 33.

That is, when centrifugal force is applied to the container 30, the liquid interface B2a of the specific gravity adjusting agent B2 held in the second holding unit 32 is located at a position that is away from the second end 33b of the flow path 33 that connects the first holding unit 31 and the second holding unit 32, as shown in Fig. 6.

Consequently, the control unit 20 adjusts the rotation speed of the motor 16 to apply an appropriate amount of centrifugal force to the blood B1, so that when the centrifugal force on the blood B1 is greater than the capillary action of the flow path 33, the blood B1 gradually moves from the first holding unit 31 to the second holding unit 32 via the flow path 33.

At this point, in the second holding unit 32, since the specific gravity adjusting agent B2 is held at a position on the inner wall 32a side that is away from the second end 33b of the flow path 33, the blood B1 that has moved to the second holding unit 32 is stacked without being mixed with the specific gravity adjusting agent B2.

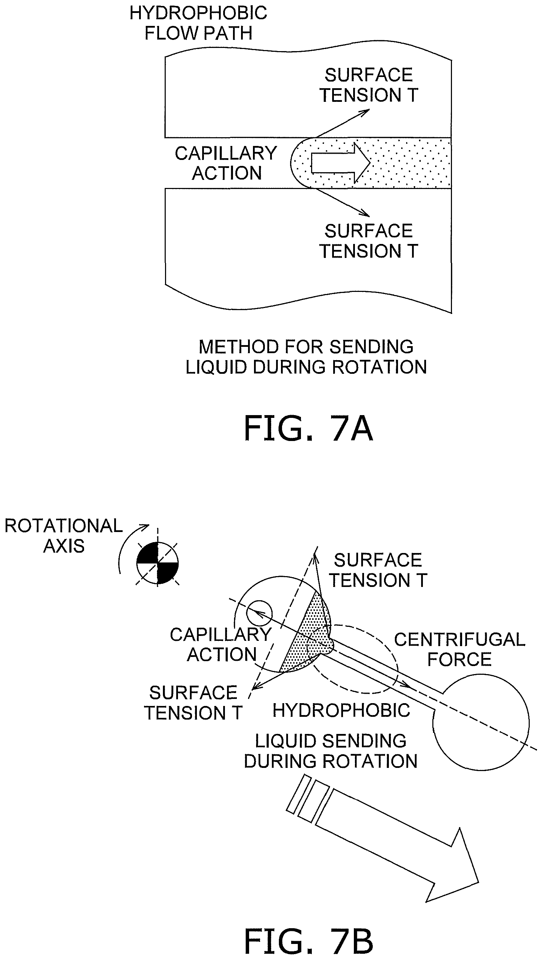

Here, the relation between the capillary action produced in the flow path 33 and the centrifugal force applied by rotation of the container 30 will be described through reference to Figs. 7A and 7B.

With a hydrophobic capillary flow path (flow path 33), once a certain rotation speed (centrifugal force) is attained, liquid feed through the flow path 33 is commenced.

This is because in the flow path 33, whose surface in contact with the blood B1 is hydrophobic, when no centrifugal force is being applied, capillary action (surface tension T) acts on the liquid, and as shown in Fig. 7A, a meniscus (curvature of the liquid surface formed by interaction with the surface of the container) that is convex to the left in the drawing is formed, and a force is generated that retains the liquid in the flow path 33.

In this state, when a centrifugal force greater than capillary action is applied to the container 30 along the direction of the flow path 33, as shown in Fig. 7B, the liquid (blood B1) in the flow path 33 moves radially outwards under this centrifugal force.

With the control unit 20 that controls the biological sample isolation device 10 in this embodiment, in the container 30 including the hydrophobic capillary flow path (flow path 33) described above, the rotation is controlled so that the proper amount of centrifugal force is applied to the container 30 in order for the blood B1 to be sent at the proper speed from the first holding unit 31 disposed on the inside in the radial direction, through the flow path 33, and to the second holding unit 32.

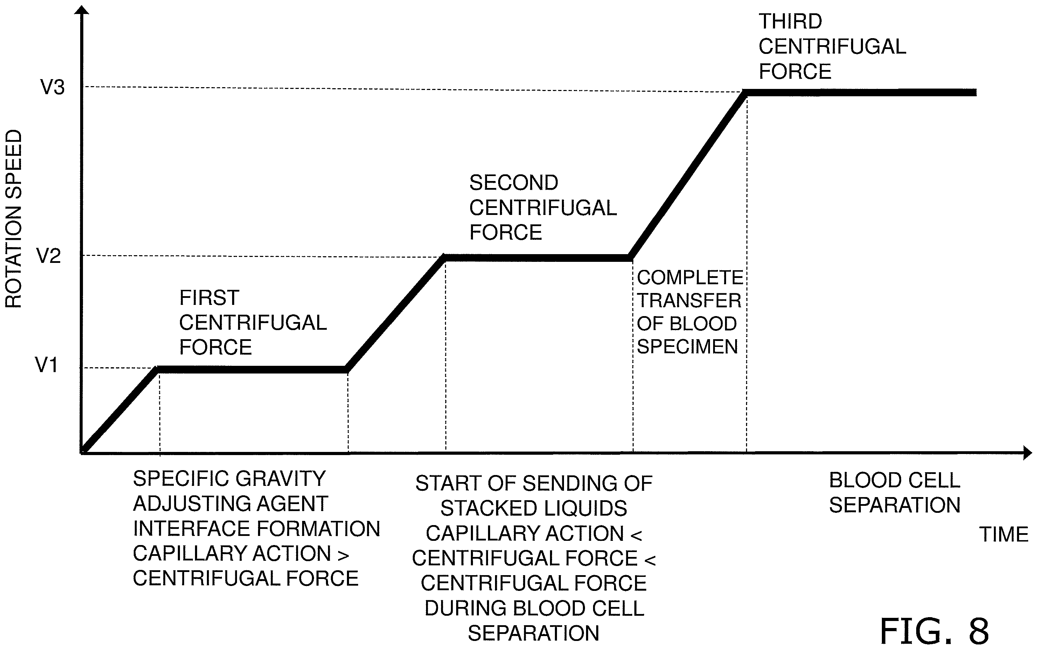

More precisely, as shown in Fig. 8, the control unit 20 sets the rotation speed for rotating the container 30 in three stages of V1, V2, and V3, and controls the motor 16 to gradually increase the rotation speed.

That is, when the container 30 is placed in the biological sample isolation device 10 and rotation control is commenced, the control unit 20 raises the rotation speed of the motor 16 to V1.

At this point, in the container 30, as shown in Fig. 6, centrifugal force forms a liquid interface B2a at a position where the specific gravity adjusting agent B2 in the second holding unit 32 is away from the second end of the flow path 33. At the same time, the blood B1 in the first holding unit 31 is gathered by centrifugal force toward the side of the inner wall 31a on the outside in the radial direction, and is held by capillary action in the flow path 33 from the first end 33a of the flow path 33.

As shown in Fig. 8, the rotation speed V1 is set so that the capillary action acting on the blood B1 in the flow path 33 and the centrifugal force (first centrifugal force) generated by rotation satisfy the following relation.

Capillary action > centrifugal force (1)

That is, the control unit 20 has a first control that controls the rotation speed of the container 30 so that a centrifugal force smaller than the capillary action generated in the flow path 33 is applied to gather the blood B1 and the specific gravity adjusting agent B2 in a state of being offset toward the radially outer surface of the first holding unit 31 and the second holding unit 32, and a second control that controls the rotation speed of the container 30 so that a centrifugal force greater than the capillary action generated in the flow path 33 is applied to send the blood B1 through the flow path 33.

Then, once the above state stabilizes, the control unit 20 increases the rotation speed of the motor 16 to V2.

At this point, in the container 30, as shown in Fig. 8, a centrifugal force that is greater than that at the rotation speed V1 starts sending the blood B1 in the first holding unit 31 a little at a time through the flow path 33 to the second holding unit 32, while the liquid interface B2a is still formed at a position where the specific gravity adjusting agent B2 in the second holding unit 32 is away from the second end of the flow path 33.

As shown in Fig. 8, the rotation speed V2 is set such that the capillary action acting on the blood B1 in the flow path 33 and the centrifugal force (second centrifugal force) generated by rotation satisfy the following relation.

Capillary action < centrifugal force < centrifugal force during blood cell isolation (2)

Then, the blood B1 is sent to through the flow path 33 the second holding unit 32 by the centrifugal force applied at the rotation speed V2, and once almost all of the blood B1 has been sent out, the control unit 20 further increases the rotation speed of the motor 16 to V3.

At this point, in the container 30, as shown in Fig. 8, the blood B1 in the first holding unit 31 is completely sent through the flow path 33 to the second holding unit 32 by a centrifugal force larger than that at the rotation speed V2 before the rotation speed changes from V2 to V3.

The rotation speed V3 is set so that a third centrifugal force used for blood cell isolation is applied, as shown

6

in Fig. 8.

That is, the control unit 20 further includes a third control that controls the rotation speed of the container 30 so that after the above-mentioned second control, the container 30 is rotated at a rotation speed higher than in the second control in a state in which the blood B1 and the specific gravity adjusting agent B2 are stacked, thus isolating a specific amount contained in the blood B1.

In this embodiment, as described above, the blood B1 gradually moves from inside the first holding unit 31 to the second holding unit 32 via the flow path 33 where capillary action acts, and during this movement of the blood B1, the specific gravity adjusting agent B2 in the second holding unit 32 is kept at a position that is away from the second end 33b of the flow path 33 through which the blood B1 is sent.

Therefore, the blood B1 does not mix with the specific gravity adjusting agent B2 in the second holding unit 32 to which it has moved, and appropriately sending the blood B1 and the specific gravity adjusting agent B2 allows a stacked state to be formed.

The rotation speeds V1, V2, and V3 are preferably set appropriately according to various conditions, such as the cross-sectional area (thickness) of the flow path 33, and the properties (viscosity, etc.) of the biological sample (blood).

Consequently, appropriately setting the rotation speed according to various conditions allows the rate at which the blood B1 is sent through the flow path 33 to be properly adjusted.

As a result, unlike when a stacked state of the blood B1 and the specific gravity adjusting agent B2 is formed by manually, variance due to human skill can be prevented, and a stacked state of the blood B1 and the specific gravity adjusting agent B2 can always be formed stably.

Biological Sample Isolation Control Method

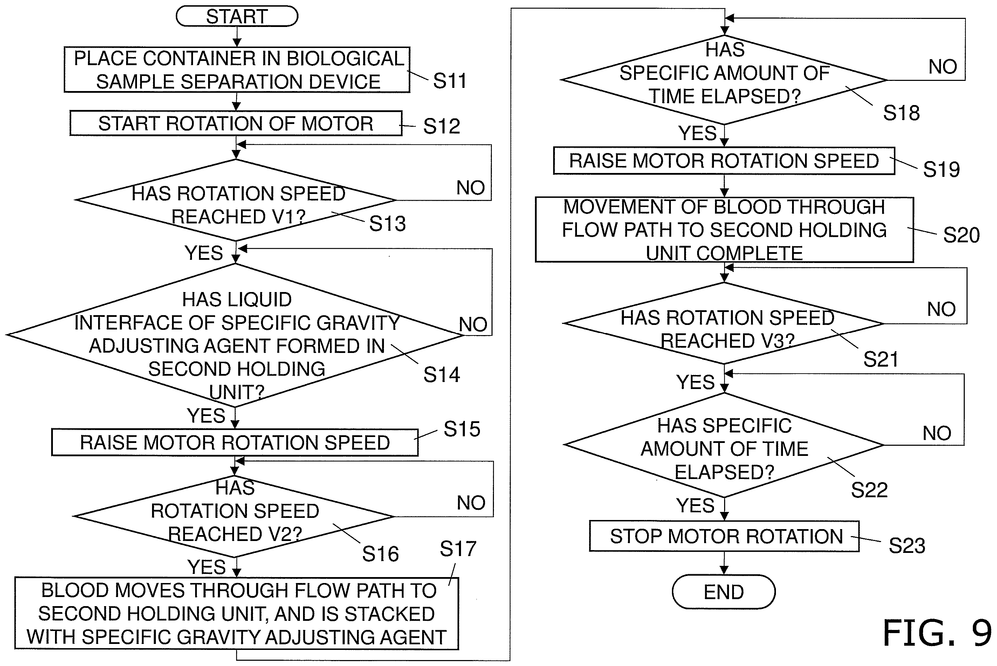

The biological sample isolation control method of this embodiment will now be described through reference to the flowchart in Fig. 9.

In this embodiment, the above-mentioned container 30 is placed the biological sample isolation device 10 and rotated to apply centrifugal force, and this stacks the blood B1 held in the first holding unit 31 of the container 30 with respect to the specific gravity adjusting agent B2 held in the second holding unit 32.

More specifically, in step S11, when the power button 15 is pressed to turn on the power to the biological sample isolation device 10, first, as shown in Fig. 2, the pullout section 12 of the biological sample isolation device 10 is pulled out, and in this state one or more containers 30 are placed on the upper surface of the container holder 12a, and the pullout section 12 is retracted to its initial position.

Next, in step S12, the control unit 20 starts rotating the motor 16 to rotate the containers 30.

Next, in step S13, until the rotation of the motor 16 speed reaches V1, it is determined whether or not V1 has been reached, and once the rotation speed reaches V1, the processing moves to step S14.

Next, in step S14, in a state in which the container 30 is rotating at the rotation speed V1, the system waits until the liquid interface of the specific gravity adjusting agent B2 in the second holding unit 32 is formed, and once this liquid interface is formed, the processing proceeds to step S15.

At this point, the blood B 1 in the first holding unit 31 enters the flow path 33 from the first end 33a of the flow path 33 and is held by capillary action (capillary action > first centrifugal force) (Fig. 8) (first step).

The presence or absence of a liquid interface of the specific gravity adjusting agent B2 in the second holding unit 32 is used as a condition for determining whether or not the blood B 1 is ready to be sent to the second holding unit 32 via the flow path 33.

That is, the formation of a liquid interface of the specific gravity adjusting agent B2 in the second holding unit 32 means that the specific gravity adjusting agent B2 is in a state of having been gathered and held by centrifugal force on the inner wall 32a on the outside in the radial direction. Consequently, in this state, even if blood B1 is sent from the flow path 33, since the second end 33b of the flow path 33 connected to the second holding unit 32 is located away from the liquid interface of the specific gravity adjusting agent B2, the blood B1 is unlikely to mix with the specific gravity adjusting agent B2.

Next, in step S15, the control unit 20 increases the rotation speed of the motor 16 from the rotation speed V1.

Next, in step S16, until the rotation speed of the motor 16 reaches V2, it is determined whether or not the rotation speed has reached V2, and once the rotation speed reaches V2, the processing moves to step S17.

Next, in step S17, since the centrifugal force increases as the rotation speed increases to V2, the centrifugal force is greater than the capillary action acting on the blood B1 in the flow path 33, and therefore the blood B1 moves through the flow path 33 to the second holding unit 32 (second step), resulting in a state of being stacked with the specific gravity adjusting agent B2 in the second holding unit 32.

Next, in step S18, the system waits until a specific amount of time has elapsed since reaching the rotation speed V2, and once this time has elapsed, the processing proceeds to step S19.

Next, in step S19, the control unit 20 increases the rotation speed of the motor 16 from the rotation speed V2.

Next, in step S20, while the rotation speed increases from V2 to V3, the blood B1 completes its movement from the first holding unit 31 to the second holding unit 32 via the flow path 33.

Next, in step S21, until the rotation speed of the motor 16 reaches V3, it is determined whether or not the rotation speed has reached V3, and once the rotation speed reaches V3, the processing moves to step S22.

Next, in step S22, the system waits until a specific amount of time has elapsed since reaching the rotation speed V3, and once this time has elapsed, the processing proceeds to step S23.

Here, the rotation speed V3 is set so as to impart a third centrifugal force to perform blood cell isolation in which a specific component is isolated from the blood B1.

Next, in step S23, the control unit 20 stops the rotation of the motor 16.

Main features

The control unit (biological sample isolation control device) 20 of this embodiment controls the biological sample isolation device 10 so as to perform processing to rotate a container 30 in which the blood B1 and the specific gravity adjusting agent B2 have been placed in isolate spaces, and thereby stack the blood B1 and the specific gravity adjusting agent B2. The container 30 has the first holding unit 31 in which the blood B1 is held, the second holding unit 32 in which the specific gravity adjusting agent B2 is held, and a hydrophobic flow path 33 that connects the first holding unit 31 and the second holding unit 32 and that is subject to capillary action. The biological sample isolation device 10 has the container holder 12a that holds the container 30 such that the first holding unit 31 and the second holding unit 32 are disposed at different distances in the radial direction from the rotational center O, and the motor 16 that rotates the container 30 around the rotational center O. The control unit 20 controls the motor 16 so that the blood B1 moves through the flow path 33 between the first holding unit 31 and the second holding unit 32, and the blood B1 and the specific gravity adjusting agent B2 are stacked.

Here, the blood B1 held in the first holding unit 31 of the container 30 is sent through the hydrophobic flow path 33 to the second holding unit 32 side where the specific gravity adjusting agent B2 is held.

At this point, a force acts on the hydrophobic flow path 33 to keep the blood B1 in the flow path 33 by capillary action.

Consequently, the rotation speed of the container 30 is controlled to apply to the blood B1 a centrifugal force that is slightly larger than the capillary action acting on the flow path 33, so that the blood B1 can be gradually sent from the first holding unit 31 to the second holding unit 32 at an appropriate fluid delivery rate, via the flow path 33 that connects the first holding unit 31 and the second holding unit 32 is controlled.

As a result, this avoids the mixing of the blood B1 and the specific gravity adjusting agent B2 in the container 30, and an appropriately stacked state can be achieved.

Other Embodiments

An embodiment of the present invention was described above, but the present invention is not limited to or by the above embodiment, and various changes are possible without departing from the gist of the invention.

(A)
In Embodiment 1 above, an example was given of realizing the present invention as the control unit 20 installed in the biological sample isolation device 10, and a biological sample isolation control method. However, the present invention is not limited to this.

For example, the present invention may be realized as a biological sample isolation control program that causes a computer to execute the above-mentioned biological sample isolation control method.

This biological sample isolation control program is stored in a memory (storage unit) installed in the biological sample isolation control device, and a CPU reads the biological sample isolation control program stored in the memory and causes the hardware to execute the various steps. More specifically, the same effect as above can be obtained by having the CPU read the biological sample isolation control program and execute the above-mentioned first step, second step, and third step.

Also, the present invention may be realized as a recording medium on which a biological sample isolation control program is stored.

(B)

In Embodiment 1 above, as shown in Fig. 6, an example was given of the configuration of the container 30 in which the flow path 33 connecting the first holding unit 31 and the second holding unit 32 was disposed along the radial direction of a circle centered on the rotational center O, the first end 33a of the flow path 33 was connected to the inner wall 31a on the outside of the first holding unit 31 in the radial direction, and the second end 33b was connected to the surface on the inside of the second holding unit 32 in the radial direction. However, the present invention is not limited to this.

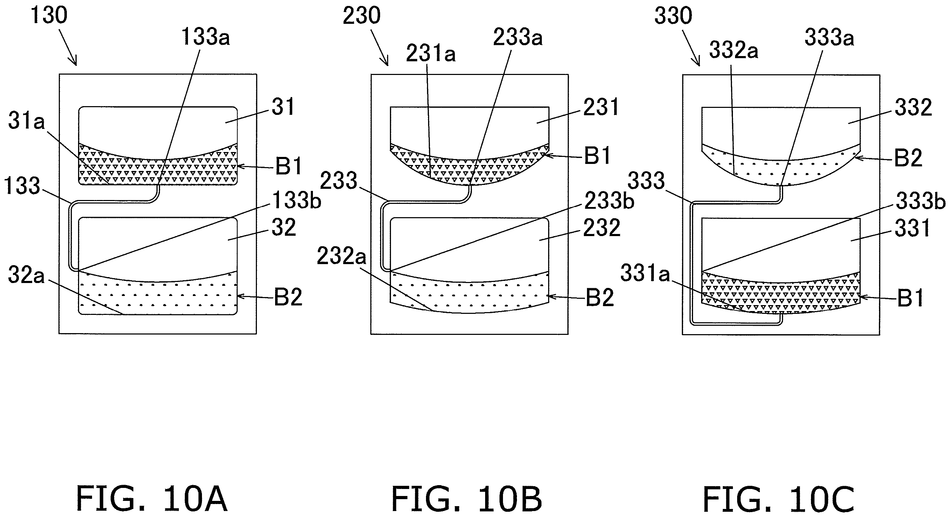

For example, as shown in Fig. 10A, the configuration of the container 30 may be such that a first end 133a of a flow path 133 connecting the first holding unit 31 and the second holding unit 32 is connected to the inner wall 31a on the outside of the first holding unit 31 in the radial direction, and a second end 133b is connected to the side surface of the second holding unit 32.

At this point, the second end 133b of the flow path 133 connected to the second holding unit 32 may to be connected to a position that is not in the liquid of the specific gravity adjusting agent B2 and is away from the liquid interface.

(C)

In Embodiment 1 above, as shown in Fig. 6, an example was given of the configuration of the container 30 in which the radially outer inner walls 31a and 32a of the first holding unit 31 and the second holding unit 32, respectively, are linear in plan view. However, the present invention is not limited to this.

For example, as shown in Fig. 10B, a container 230 may be used in which radially outer inner walls 231a and 232a of a first holding unit 231 and a second holding unit 232 are each curved.

In this case, when centrifugal force is applied, the blood B1 will tend to pool at the curved bottom portion of the first holding unit 231, so only an extremely small amount of blood B1 will remain in the first holding unit 231.

Also, at this point, the configuration may be such that the container 230 has a flow path 233 whose first end 233a is connected to the radially outer inner wall 231a of the first holding unit 231 and whose second end 233b is connected to the side surface of the second holding unit 232.

(D)

In Embodiment 1 above, as shown in Fig. 6, an example was given of the configuration of the container 30 in which the flow path 33 connecting the first holding unit 31 and the second holding unit 32 was connected to the inner wall 31a on the outside of the first holding unit 31 in the radial direction, and the second end 33b was connected to the surface on the inside of the second holding unit 32 in the radial direction. However, the present invention is not limited to this.

For example, as shown in Fig. 10C, the configuration of the container 330 may be such that a first end 333a of a flow path 333 is connected to the inner wall 332a on the outside of the second holding unit 332 in the radial direction, and a second end 333b is connected to the inner wall 331a on the outside of the first holding unit 331 in the radial direction.

At this point, the specific gravity adjusting agent B2 is held in the second holding unit 332 disposed on the radially inner side of the container 330, and the blood B1 is held in the first holding unit 331 disposed on the radial outer side. When centrifugal force is then applied, the specific gravity adjusting agent B2 can be gradually stacked from the lower layer side in the drawing (the outside in the radial direction) within the container 330 in which the second end 333b is connected to the inner wall 331a on the outside of the first holding unit 331 in the radial direction.

Also, at this point, the inner walls on the outside of the first holding unit 331 and the second holding unit 332 in the radial direction may be curved as shown in Fig. 10C in plan view, or may be linear as shown in Fig. 10A.

(E)

In Embodiment 1 above, an example was given in which blood was used as a biological sample and a specific gravity adjusting agent was used as a reagent. However, the present invention is not limited to this.

For example, the biological sample may be a sample other than blood collected from a living body, and the reagent may be a drug or additive rather than a specific gravity adjusting agent.

(F)

In Embodiment 1 above, an example was given in which the flow path 33 connecting the first holding unit 31 and the second holding unit 32 was disposed substantially parallel to the radial direction. However, the present invention is not limited to this.

For example, the flow path connecting the first holding unit and the second holding unit need not be parallel to the radial direction, and may be configured as a container disposed diagonally.

(G)

In Embodiment 1 above, an example was given in which the first holding unit 31 and the second holding unit 32 were disposed along the radial direction. However, the present invention is not limited to this.

For example, the first holding unit and the second holding unit need not be arranged along the radial direction, and may be configured as containers disposed diagonally.

Embodiment 2

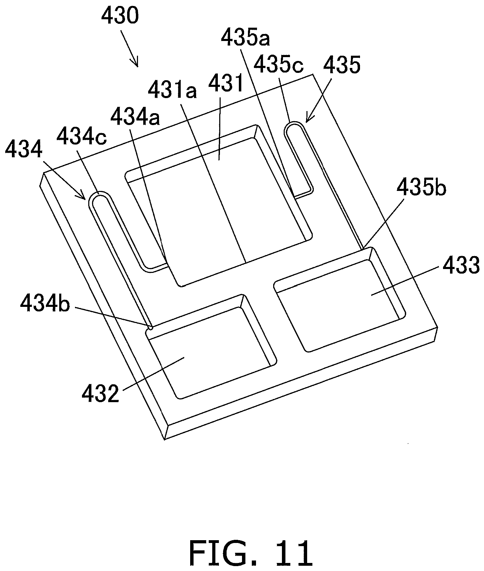

The container (biological sample isolation container) 430, control unit (biological sample isolation control device) 20, and biological sample isolation control method according to another embodiment of the present invention will now be described through reference to Figs. 11 to 17.

Those components having the same functions as in Embodiment 1 will be numbered the same and will not be described again in detail.

(1) Configuration of Biological Sample Isolation Device 10

The biological sample isolation device (rotation device) 10 according to this embodiment rotates a container (biological sample isolation container) 430 to isolate a specific component from blood B 1, and is a centrifugal isolator used to isolate and collect a specific component (mononuclear cell layer B4) of the blood B 1 from a state in which the blood B 1 is stacked so as not to mix with a specific gravity adjusting agent B2.

Here, for example, in the step of isolating mononuclear cells from blood (whole blood), it is necessary to place a specific gravity adjusting agent in a centrifuge container, and perform a centrifugal isolation process in a state in which the blood and specific gravity adjusting agent are stacked to isolate and collect a specific component (mononuclear cell layer B4). If this isolation and collection of a specific component of blood is performed manually using a pipette or the like, there is a risk that the layers may not be collected properly.

In view of this, in this embodiment, the control unit 20 controls the rotation of the container 430 by the biological sample isolation device 10 such that the specific component (mononuclear cell layer B4) can be isolated and collected from a state in which the blood B1 and the specific gravity adjusting agent are stacked in the container 430.

As shown in Fig. 1, the biological sample isolation device 10 comprises the housing unit 11, the pullout section 12, the base member 13, the display unit 14, the power button 15, the motor (rotation drive unit) 16 (see Fig. 3), the board unit 17 (see Fig. 3), and the control unit (biological sample isolation control device, control unit) 20 (see Fig. 4).

The components of the biological sample isolation device 10 here are the same as in Embodiment 1, and therefore will not be described again.

(2) Configuration of Container 430

The container 430 used to isolate and collect a specific component (mononuclear cell layer B4) of the blood B1 in the biological sample isolation device 10 of this embodiment will now be described through reference to Figs. 11 to 13.

As shown in Fig. 11, the container 430 comprises a isolation chamber (isolation unit) 431 in which the blood B1 and the specific gravity adjusting agent B2 are sealed in a stacked state, a collection chamber (collection unit) 432 that isolates and collects a specific component (mononuclear cell layer B4) of the blood B1, a waste liquid holding chamber (waste liquid holding unit) 433 that holds the component (waste liquid) other than the specific component (mononuclear cell layer B4) that is collected in the collection chamber 432, a hydrophilic first flow path 434 that connects the isolation chamber 431 and the collection chamber 432, and a hydrophilic second flow path 435 that connects the isolation chamber 431 and the waste liquid holding chamber 433.

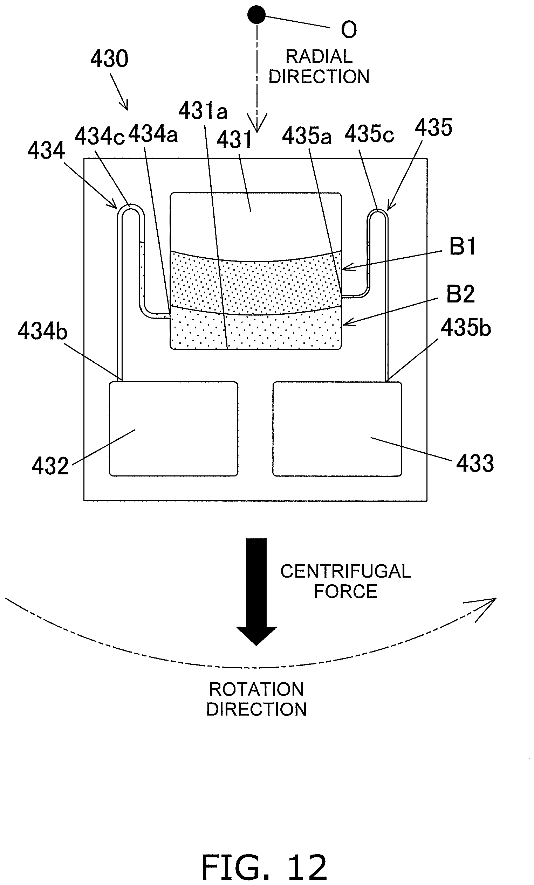

The isolation chamber 431 is disposed on the inside in the radial direction of the container 430, as shown in Fig. 11. As shown in Fig. 12, the blood B1 and the specific gravity adjusting agent B2 are sealed in the isolation chamber 431, and the blood B1 and the specific gravity adjusting agent B2 are held in a state of being stacked, without mixing with each other.

The collection chamber (collection unit) 432 is connected to the isolation chamber 431 by a hydrophilic flow path (first flow path 434) in which capillary action is produced, and is provided for isolation and collection of a specific component of the blood B1 (mononuclear cell layer B4). As shown in Fig. 12, the collection chamber 432 is disposed more to the outside in the radial direction than the rotational center O with respect to the isolation chamber 431 when placed in the biological sample isolation device 10, and collects a specific component of the blood B1 (mononuclear cell layer B4).

The waste liquid holding chamber (waste liquid holding unit) 433 is connected to the isolation chamber 431 by a hydrophilic flow path (second flow path 435) in which capillary action is produced, and is provided to collect the waste liquid of blood B1 and the specific gravity adjusting agent B2 other than the specific component of the blood B1 (mononuclear cell layer B4). As shown in Fig. 12, the waste liquid holding chamber 433 is disposed more to the outside in the radial direction than the rotational center O with respect to the isolation chamber 431 in a state of having been placed in the biological sample isolation device 10.

As shown in Fig. 12, the hydrophilic first flow path 434 connects the isolation chamber 431 and the collection chamber 432, and is a flow path in which capillary action acts. When centrifugal force is applied to the first flow path 434 by rotation in the direction of the one-dot chain arrow in Fig. 12, this force moves the specific component (mononuclear cell layer B4) of the blood B1 from the isolation chamber 431 to the collection chamber 432 by the siphon principle.

The hydrophilic second flow path 435 connects the isolation chamber 431 and the waste liquid holding chamber 433, and is a flow path in which a capillary action of a different magnitude from that of the first flow path 434 acts. When centrifugal force is applied to the second flow path 435 by rotation in the direction of the one-dot chain arrow in Fig. 12, the component that becomes waste liquid is moved from the isolation chamber 431 to the waste liquid holding chamber 433 by the siphon principle.

As discussed above, the container 430 of this embodiment is configured so that there is a difference in the capillary action produced in the first flow path 434 and the second flow path 435.

More specifically, the first flow path 434 is formed to have a larger cross-sectional area (thickness) than the second flow path 435. Consequently, the capillary action produced in the second flow path 435 is greater than the capillary action produced in the first flow path 434.

As shown in Fig. 12, substantially the same amount of centrifugal force is applied to the first flow path 434 and the second flow path 435, which are disposed at the same distance from the rotational center O in the radial direction. Because there is a difference in the capillary action produced in the first flow path 434 and the second flow path 435, the timing of the movement of the liquid through the first flow path 434 can be offset from the timing of the movement of the liquid through the second flow path 435 according to changes in the centrifugal force. As a result, the isolation and collection of the specific component (mononuclear cell layer B4) from the first flow path 434, and the collection of waste liquid from the second flow path 435 can be controlled by varying the amount of centrifugal force through control of the rotation speed of the container 430.

Furthermore, as shown in Fig. 12, the first flow path 434 has a first end 434a that is connected to the isolation chamber 431, a second end 434b that is connected to the collection chamber 432, and a return part 434c that is formed between the first end 434a and the second end 434b.

As shown in Fig. 12, the first end 434a is connected to the left side surface of the isolation chamber 431 that is substantially parallel to the radial direction. The first end 434a is connected at a position (first position) that is offset more to the outside in the radial direction than the first end 435a of the second flow path 435.

As shown in Fig. 12, the second end 434b is connected to an end surface of the collection chamber 432 that is substantially perpendicular to the radial direction.

As shown in Fig. 12, the return part 434c is a bent portion that is provided between the first end 434a and the second end 434b so as to connect the flow path (inward flow path) that exits the first end 434a and is formed facing radially inward at first, to a flow path (outward flow path) facing radially outward.

Similarly, as shown in Fig. 12, the second flow path 435 has a first end 435a that is connected to the isolation chamber 431, a second end 435b that is connected to the collection chamber 432, and a return part 435c that is formed between the first end 435a and the second end 435b.

As shown in Fig. 12, the first end 435a is connected to the right side surface of the isolation chamber 431 that is substantially parallel to the radial direction. The first end 435a is connected at a position (second position) that is offset more to the inside in the radial direction than the first end 434a of the first flow path 434.

That is, the first flow path 434 is connected to the isolation chamber 431 at a position (first position) that is more to the outside in the radial direction than the second flow path 435.

As shown in Fig. 12, the second end 435b is connected to an end surface of the waste liquid holding chamber 433 that is substantially perpendicular to the radial direction.

As shown in Fig. 12, the return part 435c is a bent portion that is provided between the first end 435a and the second end 435b so that the flow path that exits the first end 435a and is formed facing radially inward at first is changed to face radially outward. As shown in Fig. 12, the return part 435c is provided at a position that is substantially in left and right symmetry around the isolation chamber 431 with respect to the return part 434c on the first flow path 434 side.

Also, as shown in Fig. 12, the return part 435c on the first flow path 434 side and the return part 435c on the second flow path 435 side are disposed at a position that is more to the inside in the radial direction than the liquid surface where the blood B1 and the specific gravity adjusting agent B2 are stacked in the step of rotating the container 430 to isolate and collect the specific component of the blood B1.

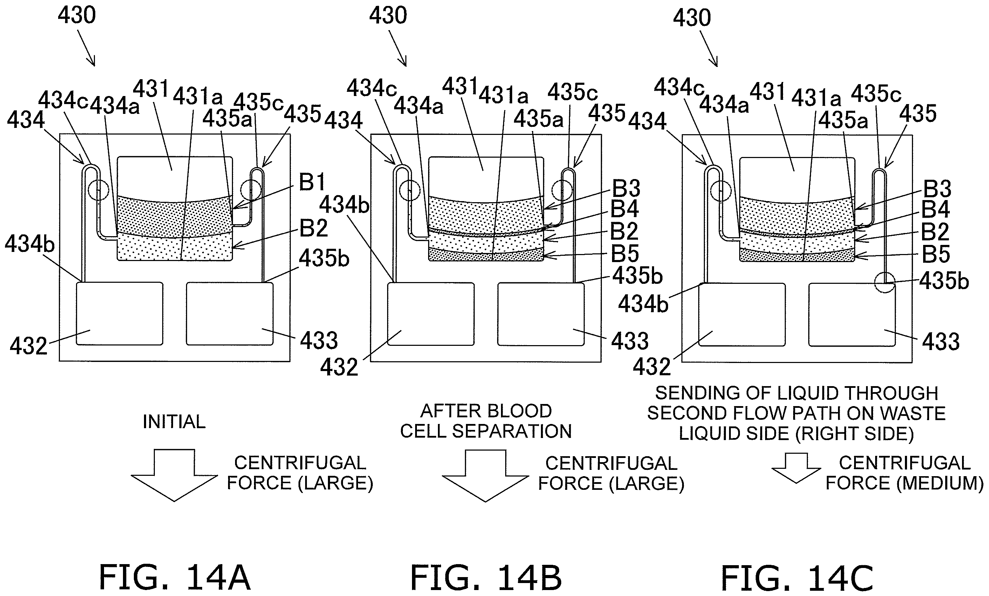

Furthermore, with the container 430 of this embodiment, the first position where the first flow path 434 is connected to the isolation chamber 431, and the second position where the second flow path 435 is connected to the isolation chamber 431 are disposed so as to sandwich the layer containing the specific component of the blood B1 (mononuclear cell layer B4) in the radial direction in a state in which centrifugal force is applied (see Fig. 14B).

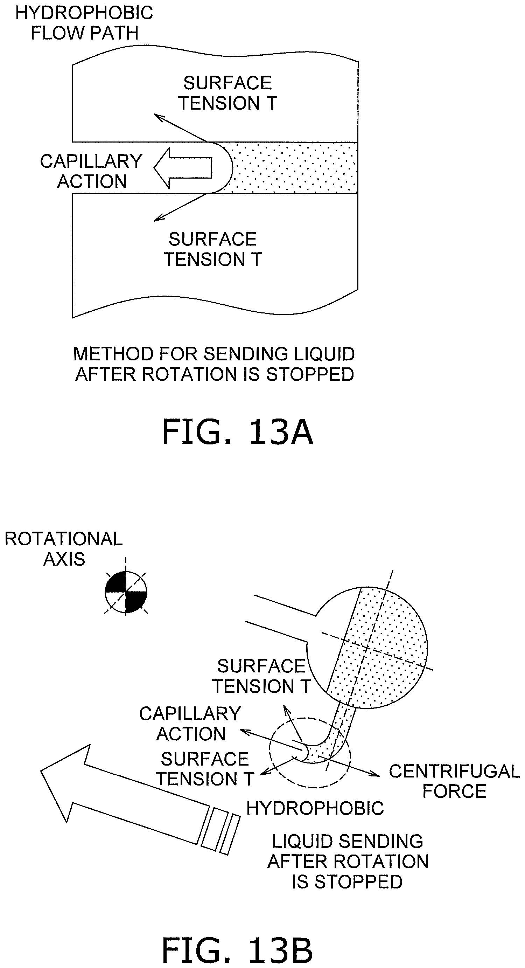

Here, the relation between the capillary action produced in the first flow path 434 and the second flow path 435 and the centrifugal force applied by rotating the container 430 will be described through reference to Figs. 13A and 13B.

With a hydrophilic capillary flow path (first flow path 434 and second flow path 435), when the rotation speed (centrifugal force) drops below a certain level, the liquid begins to flow through the first flow path 434 and the second flow path 435.

This is because in a state in which no centrifugal force is being applied to the first flow path 434 and the second flow path 435, whose surfaces in contact with the blood B1 are hydrophilic, capillary action (surface tension T) acts on the liquid, and as shown in Fig. 13A, a concave state is formed on the left side in the figure, and in a state in which centrifugal force is applied, a force is generated that keeps the liquid in the first flow path 434 and the second flow path 435.

From this state, as shown in Fig. 13B, when the centrifugal force on the container 430 becomes less than the capillary action along direction of the first flow path 434 and the second flow path 435, the liquid (blood B1) in the flow path 434 and the second flow path 435 moves radially inward due to capillary action. This makes it possible to send the liquid (blood B1) in the first flow path 434 and the second flow path 435 while the rotation is stopped.

With the control unit 20 that controls the biological sample isolation device 10 of this embodiment, in this container 430 including the hydrophilic capillary flow paths (the first flow path 434 and the second flow path 435), rotation control is performed to change the amount of centrifugal force applied to the container 430 in order to send the specific component or waste liquid at the appropriate timing from the isolation chamber 431 disposed on the inside in the radial direction, through the first flow path 434 and the second flow path 435, to the collection chamber 432 and the waste liquid holding chamber 433 disposed on the outside in the radial direction.

More specifically, first, when the container 430 is rotated at a specific rotation speed by the biological sample isolation device 10, centrifugal force (large) is applied to the blood B1 and the specific gravity adjusting agent B2 held in the isolation chamber 431 of the container 430, as shown in Fig. 14A. At this point, the blood B1 and the specific gravity adjusting agent B2 are pushed by centrifugal force toward the inner wall 431a on the outside of the isolation chamber 431 in the radial direction, and are held in a stacked state, without mixing with each other. At this point, the liquid level in the first flow path 434 and the second flow path 435 is kept at approximately the same height (in the radial direction; see the dotted circle in the drawing).

Next, if the rotation of the container 430 is continued at the same speed (centrifugal force (large)) in the state shown in Fig. 14A, the blood cells are isolated from the state in which the blood B1 and the specific gravity adjusting agent B2 are stacked, and the layers of plasma B3, mononuclear cell layer (specific component) B4, specific gravity adjusting agent B2, and red blood cells B5 are formed, in that order, from the inside in the radial direction.

Next, if the rotation speed of the container 430 is lowered to a specific level, the centrifugal force (large) applied to the liquid in the container 430 decreases to a centrifugal force (medium) as shown in Fig. 14C, and this sends the liquid in the second flow path 435 on the side where the capillary action is larger (the cross-sectional area is smaller). At this point, in the second flow path 435, as shown in Fig. 14C, waste liquid (plasma B3), which has the lowest specific gravity in the isolation chamber 431 is sent, which moves the liquid level to the position of connection to the waste liquid holding chamber 433 (second end 435b; see the dotted circle in the drawing).

The relation between capillary action and centrifugal force in the state of Fig. 14C is expressed by the following relational expression (1).

Capillary action in first flow path 434 < centrifugal force < capillary action in second flow path 435        (1)

That is, the rotation speed at which the container 430 is rotated in the state shown in Fig. 14C is set so as to satisfy the above relational expression (1) according to the cross-sectional area of the first flow path 434 and the second flow path 435, etc.

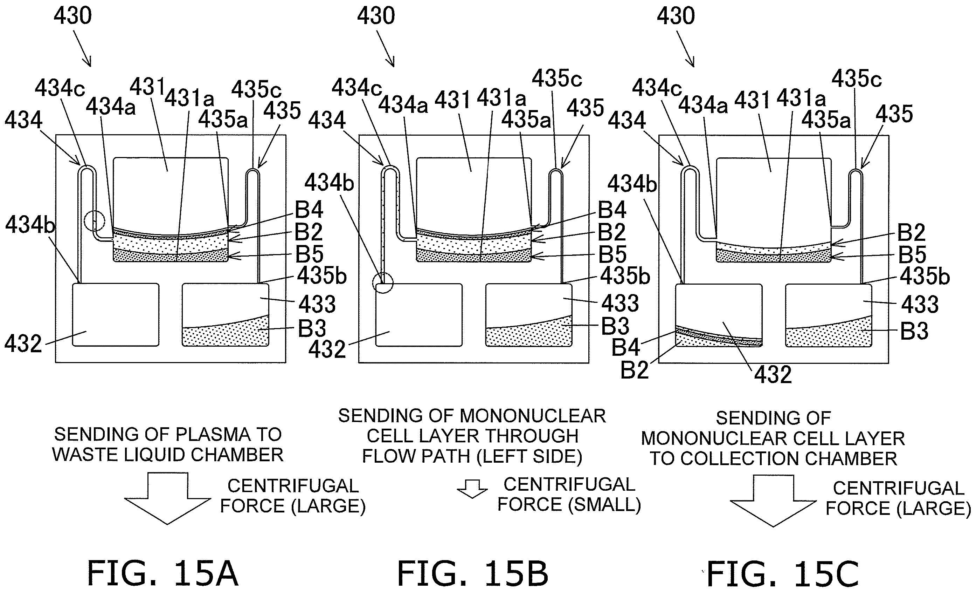

Next, if the rotation speed of the container 430 is increased to a specific level again, as shown in Fig. 15A, the centrifugal force (medium) applied to the liquid in the container 430 increases to the centrifugal force (large). Consequently, the plasma B3 sent from the second end 435b of the second flow path 435 to the waste liquid holding chamber 433 is sent in a state in which the delivery speed has been raised by the increase in centrifugal force. Furthermore, since the inside of the second flow path 435 is filled with liquid, siphoning occurs, and all of the liquid up to the position where the first end 435a is connected in the isolation chamber 431 is sent.

At this point, in the second flow path 435, as shown in Fig. 15A, the plasma B3 up to a position more to the outside than the first end 435a in the radial direction is sent from inside the isolation chamber 431 to the waste liquid holding chamber 433 via the second flow path 435.

This causes almost all of the plasma B3 in the isolation chamber 431 to be sent to the waste liquid holding chamber 433.

Also, at this point, since the plasma B3 is sent to the waste liquid holding chamber 433 and the liquid level in the isolation chamber 431 moves radially outward, the liquid level held in the first flow path 434 also moves to about the same position.

Next, if the rotation speed of the container 430 is lowered to a specific level again, the centrifugal force (large) applied to the liquid in the container 430 decreases to the centrifugal force (small), as shown in Fig. 15B. This causes the remaining plasma B3 and the mononuclear cell layer B4, which have the highest specific gravity remaining in the isolation chamber 431, to be sent from the second end 434b of the first flow path 434 into the collection chamber 432.

At this point, the centrifugal force (small) applied to the liquid in the isolation chamber 431 is even smaller than the centrifugal force (medium) when sending the waste liquid shown in Fig. 14C. The relation between capillary action and centrifugal force in the state of Fig. 15B is expressed by the following relational expression (2).

$$\text{Capillary action in first flow path 434} > \text{centrifugal force} \ \dots (2)$$

That is, capillary action becomes dominant when the rotation of the container 430 is controlled so that the centrifugal force (small) applied to the liquid in the isolation chamber 431 is smaller than the capillary action produced in the first flow path 434. Consequently, the liquid is sent through the first flow path 434, and the liquid level moves to the position of the connection portion (second end 434b) with the collection chamber 432 (see the dotted circle in the figure).

Next, if the rotation speed of the container 430 is increased to a specific level again, the centrifugal force (small) applied to the liquid in the container 430 increases to the centrifugal force (large) as shown in Fig. 15C, which causes the mononuclear cell layer B4 sent from the second end 434b into the collection chamber 432 while increasing the liquid delivery speed through an increase in centrifugal force.

At this point, in the first flow path 434, as shown in Fig. 15C, the mononuclear cell layer B4, the specific gravity adjusting agent B2, etc., up to a position more to the outside than the first end 434a in the radial direction are sent from the isolation chamber 431 to the collection chamber 432 via the first flow path 434.

The relation between capillary action and centrifugal force in the state of Fig. 15C is expressed by the following relational expression (3).

$$\text{Capillary action of first flow path } 434 > \text{centrifugal force} \quad \ldots (3)$$

Consequently, the liquid (mononuclear cell layer B4, etc.) held in the first flow path 434 moves radially outward under centrifugal force. Furthermore, since the inside of the first flow path 434 is filled with liquid, siphoning occurs, and all the liquid up to the position where the first end 434a is connected in the isolation chamber 431 is sent.

Biological Sample Isolation Control Method

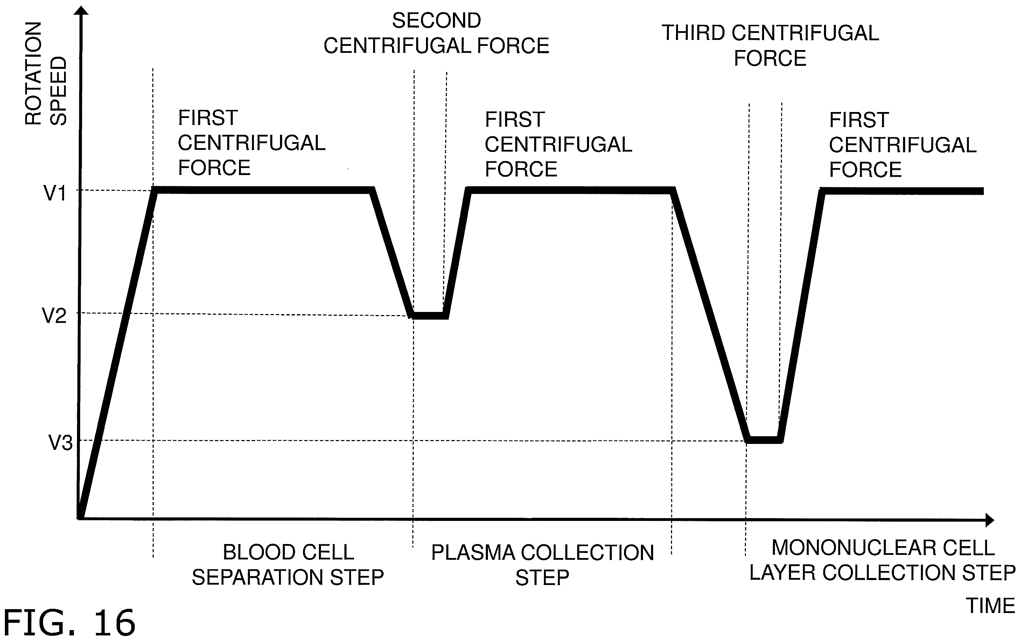

With the biological isolation control method of this embodiment, as shown in Fig. 16, the control unit 20 controls the motor 16 so as to vary the centrifugal force applied to the isolated components of the blood B1 by setting the rotation speed at which the container 430 is rotated in three stages of V1, V2, and V3, and by changing the rotation speed.

That is, in a state in which the container 430 has been placed in the container holder 12a of the pullout section 12 of the biological sample isolation device 10, the control unit 20 rotates the motor 16 and increases the rotation speed to V1 as shown in Fig. 16.

When the rotation speed V1 is reached, a first centrifugal force is applied to the liquid (blood B1, etc.) in the container 430. Then, after a specific length of time has elapsed in this state, blood cell isolation proceeds from a state in which the blood B1 and the specific gravity adjusting agent B2 in the isolation chamber 431 are stacked (blood cell isolation step).

Next, once the specific length of time has elapsed, the control unit 20 determines that blood cell isolation is complete, and reduces the rotation speed of the motor 16 from V1 to V2, as shown in Fig. 16.

At this point, when the rotation speed V2 is reached, a second centrifugal force is applied to the liquid in the container 430. The relation between capillary action and the second centrifugal force is expressed by the above-mentioned relational expression (1).

Capillary action in first flow path 434 < centrifugal force < capillary action in second flow path 435       (1)

Consequently, the waste liquid (plasma B3) with the lowest specific gravity is transferred from inside the isolation chamber 431, and the liquid level moves to the position of the connection portion with the waste liquid holding chamber 433 (see the dotted circle in the figure) as shown in Fig. 14C.

Next, as shown in Fig. 16, when the control unit 20 again increases the rotation speed of the container 430 from V2 to V1, the centrifugal force applied to the liquid in the container 430 increases from the second centrifugal force to the first centrifugal force.

Consequently, the plasma B3 sent from the second end 435b of the second flow path 435 is sent while the delivery speed is raised due to the increase in centrifugal force. Furthermore, since the inside of the second flow path 435 is filled with liquid, siphoning occurs, and all of the liquid up to the position where the first end 435a is connected in the isolation chamber 431 is sent (plasma collection step).

Next, as shown in Fig. 16, when the control unit 20 decreases the rotation speed of the container 430 from V1 to V3 again, the centrifugal force applied to the liquid in the container 430 decreases from the first centrifugal force to the third centrifugal force. Consequently, the remaining plasma B3 and the mononuclear cell layer B4, which have the highest specific gravity remaining in the isolation chamber 431, are sent from the second end 434b of the first flow path 434 into the collection chamber 432.

At this point, the relation between the third centrifugal force applied to the liquid in the isolation chamber 431 and capillary action is expressed by the following relational expression (2), as mentioned above.

$$\text{Capillary action in first flow path 434} > \text{centrifugal force} \dots (2)$$

That is, capillary action becomes dominant when the rotation of the container 430 is controlled so that the third centrifugal force applied to the liquid in the isolation chamber 431 is smaller than the capillary action produced in the first flow path 434. This causes the liquid to be sent to the first flow path 434 as shown in Fig. 15B, and the liquid level to move to the position of the connection portion (second end 434b) with the collection chamber 432 (see the dotted line circle in the drawing).

Next, as shown in Fig. 16, when the control unit 20 again increases the rotation speed of the container 430 from V3 to V1, the third centrifugal force applied to the liquid in the container 430 increases to the first centrifugal force. Consequently, the mononuclear cell layer B4 sent from the second end 434b is sent from the second end 434b into the collection chamber 432 while the liquid delivery speed is raised by the increase in centrifugal force (mononuclear cell layer collection step).

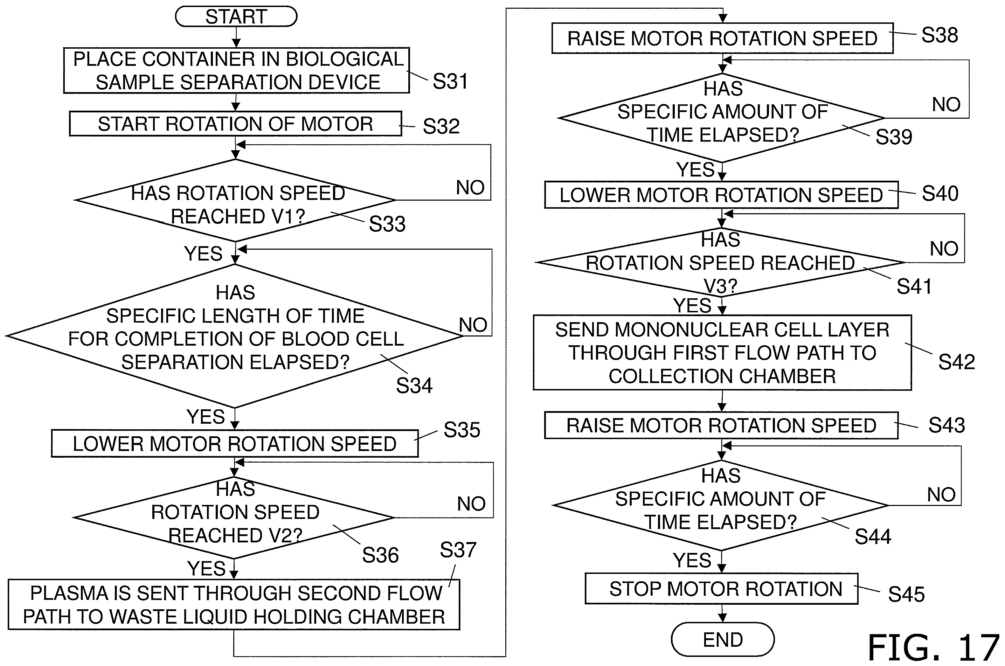

Here, the biological sample isolation control method of this embodiment will be described as follows through reference to the flowchart shown in Fig. 17.

In step S31, in the biological sample isolation device 10 in which the power button 15 has been pressed to turn on the power, first, as shown in Fig. 2, one or more containers 430 are placed on the upper surface of the portion 12a in a state in which the pullout section 12 of the biological sample isolation device 10 has been pulled out, and the pullout section 12 is then retracted to its initial position.

Next, in step S32, the control unit 20 starts rotating the motor 16 to rotate the container 430.

Next, in step S33, until the rotation speed of the motor 16 reaches V1, it is determined whether or not the rotation speed has reached V1, and once the rotation speed reaches V1, the processing moves to step S34.

Next, in step S34, in a state in which the container 430 is rotating at the rotation speed V1, the system waits until the specific length of time necessary to complete the progress of the blood cell isolation from a state where the blood B1 and the specific gravity adjusting agent B2 are stacked in the isolation chamber 431 has elapsed (first step). Then, once the specific length of time has elapsed, the processing proceeds to step S15.

Next, in step S35, the control unit 20 lowers the rotation speed of the motor 16 from the rotation speed V1.

Next, in step S36, until the rotation speed of the motor 16 reaches V2, it is determined whether or not the rotation speed has decreased, and once the rotation speed reaches V2, the processing moves to step S37.

Next, in step S37, since the second centrifugal force applied at the rotation speed V2 becomes smaller than the capillary action produced in the second flow path 435, the plasma B3 is sent through the second flow path 435 to the waste liquid holding chamber 433 (second step).

Next, in step S38, the control unit 20 again raises the rotation speed of the motor 16 from V2 to V1 (third step). This increases the speed at which the plasma B3 is sent to the waste liquid holding chamber 433 via the second flow path 435, and thus shortens the liquid delivery time.

Next, in step S39, the system waits until a specific length of time has elapsed, during which nearly all of the plasma B3 is sent from inside the isolation chamber 431, and once this specific length of time has elapsed, the processing proceeds to step S40.

Next, in step S40, the control unit 20 lowers the rotation speed of the motor 16 from V1.

Next, in step S41, until the rotation speed of the motor 16 reaches V3, it is determined whether or not the rotation speed has decreased, and once the rotation speed reaches V3, the processing moves to step S42.

Next, in step S42, since the third centrifugal force applied at the rotation speed V3 is smaller than the capillary action produced in the first flow path 434, the mononuclear cell layer B4 is sent through the first flow path 434 to the collection chamber 432 (fourth step).

In step S43, the control unit 20 again raises the rotation speed of the motor 16 from V3 to V1 (fifth step). This increases the speed at which the liquid including the mononuclear cell layer B4 is sent to the collection chamber 432 via the first flow path 434, and thus shortens the liquid delivery time.

Next, in step S44, the system waits until a specific length of time has elapsed, during which nearly all of the mononuclear cell layer B4 is sent from inside the isolation chamber 431, and once this specific length of time has elapsed, the processing proceeds to step S45.

Next, in step S45, the control unit 20 stops the rotation of the motor 16.

Main Features

The container 430 in this embodiment rotates while placed in the biological sample isolation device 10, and specific components contained in the blood B1 are individually collected. The container 430 comprises the isolation chamber 431, the collection chamber 432, the waste liquid holding chamber 433, the hydrophilic first flow path 434, and the hydrophilic second flow path 435. The isolation chamber 431 holds the blood B1 and the specific gravity adjusting agent B2. The collection chamber 432 is disposed more to the outside than the rotational center O in the radial direction with respect to the isolation chamber 431 in a state of being placed in the biological sample isolation device 10, and collects a specific component of the blood B1. The waste liquid holding chamber 433 is disposed more to the outside than the rotational center O in the radial direction with respect to the isolation chamber 431 in a state of being placed in the biological sample isolation device 10, and holds the waste liquid of the blood B1 and the specific gravity adjusting agent B2 other than the specific component. The first flow path 434 connects the isolation chamber 431 and the collection chamber 432, and when capillary action acts and a centrifugal force is applied, the specific component is moved from the isolation chamber 431 to the collection chamber 432 by the siphon principle. The second flow path 435 connects the isolation chamber 431 and the waste liquid holding chamber 433, and when capillary action having a different magnitude from that of the first flow path 434 acts and centrifugal force is applied, the waste liquid is moved from the isolation chamber 431 to the waste liquid holding chamber 433 by the siphon principle. The first flow path 434 is connected at a first position on a first side surface of the isolation chamber 431, the second flow path 435 is connected at a second position on a second side surface of the isolation chamber 431 on the opposite side from the first side, and the first position and the second position are provided at positions offset in the radial direction.

That is, with the container 430 of this embodiment, as discussed above, the first flow path 434 and the second flow path 435 are disposed so that capillary action acts in the opposite direction from the vector on which the centrifugal force acts.

Consequently, the liquid level height in the siphon flow path changes depending on the balance between the capillary action produced in the first flow path 434 and the second flow path 435 and the centrifugal force applied by the rotation of the container 430.

Then, by lowering the rotation speed (centrifugal force) until capillary action becomes the dominant condition, and maintaining the liquid level by centrifugal force, the siphon flow path comes into play and allows the liquid to be sent while maintaining the layers after blood cell isolation.

Here, the capillary action can be controlled by the flow path sizes (cross-sectional areas) of the first flow path 434 and the second flow path 435. Consequently, by connecting two flow paths (first flow path 434 and second flow path 435) having different capillary actions to the isolation chamber 431, and setting the siphon flow paths to operate with different centrifugal forces, just the layer (mononuclear cell layer B4) positioned between the first position and the second position where the two flow paths (first flow path 434 and second flow path 435) are connected can be isolated and collected.

As a result, depending on the configuration of the container 430, it is possible to collect the liquid in the siphon flow path while applying an appropriate amount of centrifugal force, and just the component to be isolated and collected can be selected and accurately collected according to the specific gravity of each component isolated from the blood B1.

Other Embodiments

An embodiment of the present invention was described above, but the present invention is not limited to Embodiment 2 above, and various changes are possible without departing from the gist of the invention.

(A)
In Embodiment 2, an example was given in which the present invention was realized as the control unit 20 installed in the biological sample isolation device 10 and a biological sample isolation control method. However, the present invention is not limited to this.

For example, the present invention may be realized as a biological sample isolation control program that causes a computer to execute the biological sample isolation control method described above.

This biological sample isolation control program is stored in a memory (storage unit) installed in the biological sample isolation control device, and a CPU reads the biological sample isolation control program stored in the memory and causes the hardware to execute the various steps. More specifically, the same effect as above can be obtained by having the CPU read the biological sample isolation control program and execute the above-mentioned first to fifth steps.

Also, the present invention may be realized as a recording medium on which a biological sample isolation control program is stored.

(B)
In Embodiment 2, as shown in Fig. 16, an example was given in which, in sending the liquid while controlling the rotation of the motor 16 at the rotation speed V1, the control unit 20 lowers the rotation speed to V2 and V3, after which it again raises the rotation speed to V1, thereby shortening the liquid delivery time. However, the present invention is not limited to this.

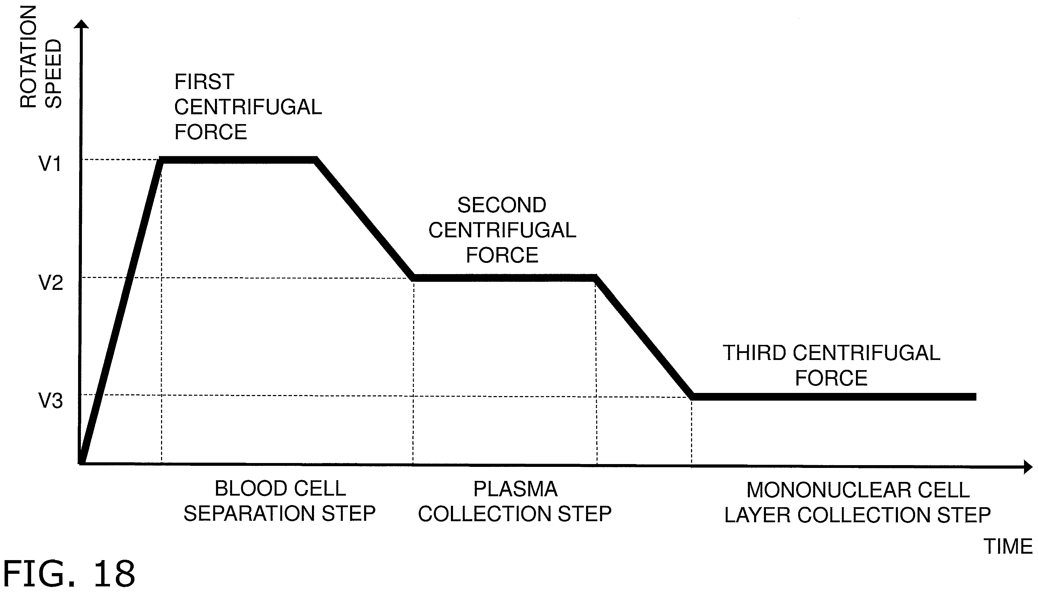

For example, as shown in Fig. 18, the control unit 20 may control the motor 16 such that after the motor 16 has rotated at the rotation speed V1 for a specific amount of time (blood cell isolation step), the rotation speed is reduced to V2 to send plasma to the waste fluid holding chamber (plasma collection step), and the rotation speed is then reduced to V3 to send the mononuclear cell layer to the collection chamber (mononuclear cell layer collection step).

In this case, compared to the rotation control shown in Fig. 16, the centrifugal force applied during liquid transfer is smaller, so the liquid delivery time is correspondingly longer, but an advantage is that a specific component of the blood (the mononuclear cell layer) can be isolated and collected in the same manner as above.

(C)

In Embodiment 2, as shown in Fig. 16, an example was given in which, when the control unit 20 lowered the rotation speed to V2 and V3 to start sending the plasma B3 and the mononuclear cell layer B4, the rotation speed of the motor 16 was again increased to V1 so as to apply the first centrifugal force. However, the present invention is not limited to this.

For example, the centrifugal force (rotation speed) applied for accelerating liquid delivery does not need to be constant, and the rotation speed of the motor may be controlled such that different amounts of centrifugal force (rotation speed) are applied in the blood cell isolation step, plasma collection step, and mononuclear cell layer collection step.

(D)

In Embodiment 2, an example was given in which the collection chamber 432 was disposed on the left side in the drawing and the container 430 on the right side, as shown in Fig. 12, etc. However, the present invention is not limited to this.

For example, the container may be configured such that the collection chamber and the waste liquid holding chamber are switched from left to right.

Here again, the same effect as above can be obtained by setting the cross-sectional area (thickness) larger on the collection chamber side and smaller on the waste liquid holding chamber side so that the cross-sectional areas of the connected flow paths will match the order of liquid delivery.

(E)

In Embodiment 2, an example was given in which, because the specific gravity of the plasma B3 sent to the waste liquid holding chamber 433 was low, the plasma B3 was first sent to the waste liquid holding chamber 433, after which the mononuclear cell layer B4 to be collected was sent. However, the present invention is not limited to this.

For example, if the specific gravities of the component that will be waste liquid and the component to be recovered are reversed, that is, if the specific gravity of the component to be recovered is lower than that of the component that will become the waste liquid, then the disposition of the collection chamber and the waste liquid holding chamber may be the opposite of that in the above embodiment.

(F)

In Embodiment 2, an example was given in which blood was used as a biological sample and a specific gravity adjusting agent was used as a reagent. However, the present invention is not limited to this.

For example, the biological sample may be a sample other than blood collected from a living body, and the reagent may be a drug, additive, or the like other than a specific gravity adjusting agent.

Embodiment 3

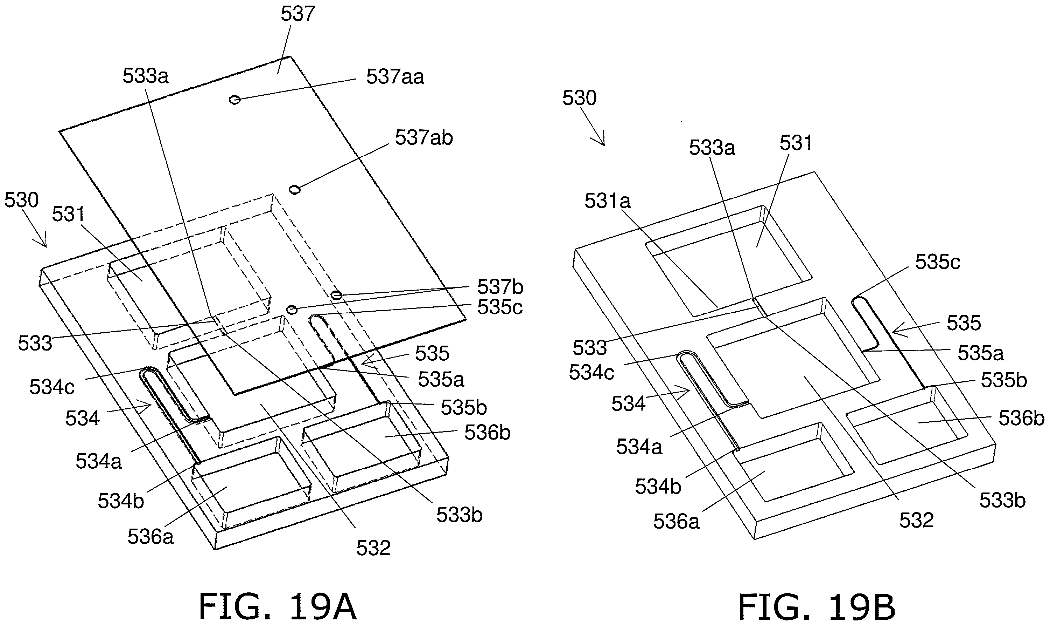

A container (biological sample isolation container) 530 according to yet another embodiment of the present invention will now be described through reference to Figs. 19A to 23.

Components having the same function as in Embodiment 1 above will be numbered the same and will not be described again in detail.

Since the components of the biological sample isolation device 10 are the same as in Embodiment 1 above, they will not be described here.

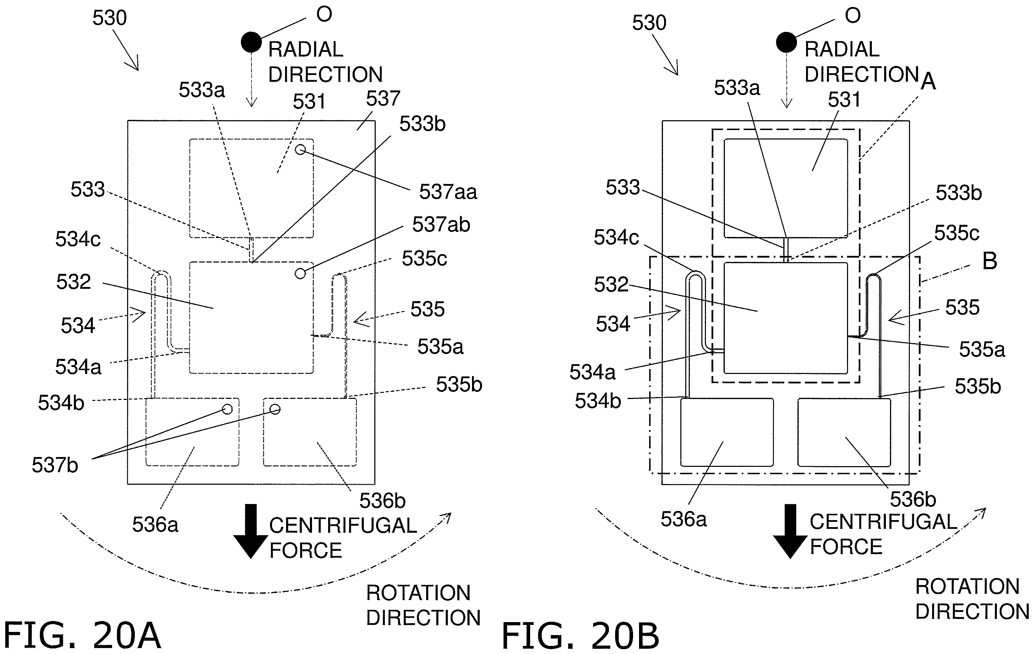

The configuration of the container 530 in this embodiment is a combination of the components on the upstream side of the container 30 of Embodiment 1 above (the first holding unit 31, the second holding unit 32, etc.; portion A in Fig. 20B), and the components on the upstream side of the container 430 of Embodiment 2 above (the isolation chamber 431, the collection chamber 432, the waste liquid holding chamber 433, etc.; portion B in Fig. 20B).

The container 530 used to isolate and collect a specific component (mononuclear cell layer B4) of blood B1 in the biological sample isolation device 10 in this embodiment is described in more specific terms below.

For the sake of explanation, injection holes 537aa and 537ab and an air hole 537b are not depicted in Fig. 19B, Fig. 20B, Fig. 21, and Fig. 22.

The container 530 is used in a state in which its front side is covered by a cover 537, as shown in Figs. 19A and 19B. The container 530 comprises a blood holding chamber (biological sample holding unit) 531 that is disposed on the innermost side in the radial direction centered on the rotational center O (discussed below) and holds blood (biological sample) B1, a isolation chamber (isolation unit) 532 in which a specific gravity adjusting agent B2 is sealed, a hydrophobic flow path 533 that connects the blood holding chamber 531 and the isolation chamber 532, a collection chamber (collection unit) 536a in which a specific component of the blood B1 (mononuclear cell layer B4), etc., is isolated and collected, a waste liquid holding chamber (waste liquid holding unit) 536b that holds components (waste liquid) other than the specific component (mononuclear cell layer B4) collected in the collection chamber 536a, a hydrophilic first flow path 534 that connects the isolation chamber 532 and the collection chamber 536a, and a hydrophilic second flow path 535 that connects the isolation chamber 532 and the waste liquid holding chamber 536b.

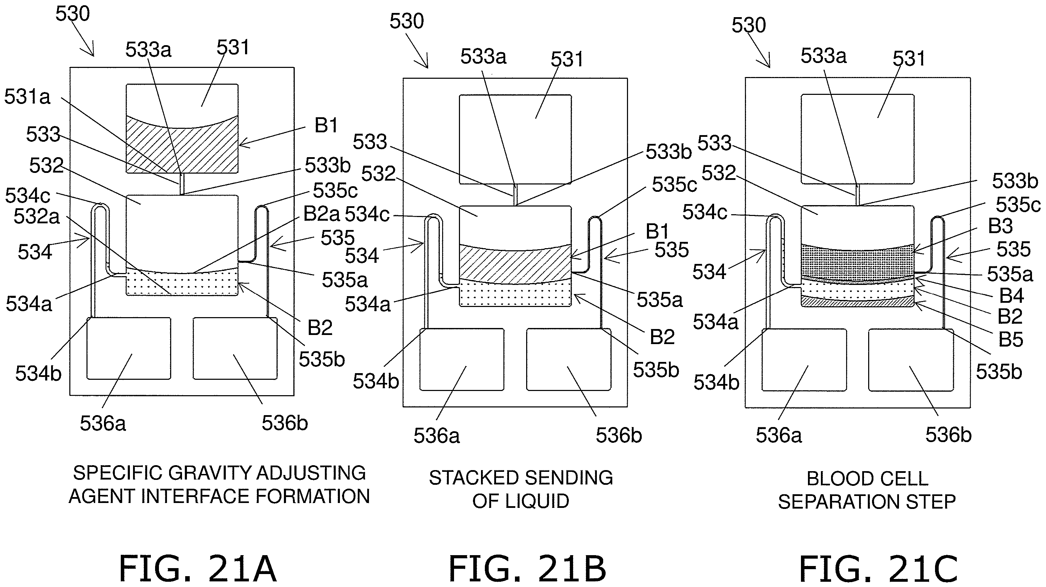

As shown in Fig. 19B, the blood holding chamber (biological sample holding unit) 531 is disposed on the innermost side in the radial direction of the container 530, and the blood B1 is sealed therein (see Fig. 21A). The blood B1 held in the blood holding chamber 531 is injected from an injection hole 537aa provided in the cover 537, as shown in Fig. 20A.

As shown in Fig. 19B, the isolation chamber 532 is disposed adjacent to the blood holding chamber 531 on the outside in the radial direction. The specific gravity adjusting agent B2 is sealed inside the isolation chamber 532 (see Fig. 21A). The specific gravity adjusting agent B2 held in the isolation chamber 532 is injected from an injection hole 537ab provided in the cover 537, as shown in Fig. 20A.

Also, the blood B1 is sent from the blood holding chamber 531 to the isolation chamber 532 in a stacked liquid delivery step (discussed below), and the blood B1 and the specific gravity adjusting agent B2 are held in a stacked state, without mixing with each other (Fig. 21B).

As shown in Fig. 20B, the blood holding chamber 531 and the isolation chamber 532 are disposed along the radial direction in a state in which the container 530 has been placed in the container holder 12a of the biological sample isolation device 10.

The hydrophobic flow path 533 is a groove formed on the upper surface of the container 530, and is formed as a flow path that produces capillary action. The flow path 533 has a first end 533a that is connected to the inner wall 531a on the outside of the blood holding chamber 531 in the radial direction and communicates with the space of the blood holding chamber 531, and a first end 533a that is connected to the inner wall on the inside of the isolation chamber 532 in the radial direction and communicates with the space of the blood holding chamber 531. Also, the flow path 533 is disposed along the radial direction in a state in which the container 530 has been placed in the container holder 12a of the biological sample isolation device 10, as shown in Fig. 20B.

Here, a case in which the motor 16 of the biological sample isolation device 10 is rotated in a state in which the blood B1 is held in the blood holding chamber 531 and the specific gravity adjusting agent B2 is held in the isolation chamber 532 will be described using Figs. 21A and 21B.

At this point, the blood B1 held in the blood holding chamber 531 of the container 530 and the specific gravity adjusting agent B2 held in the isolation chamber 532 are each subjected to centrifugal force toward the outside in the radial direction as shown in Fig. 20B.

Consequently, inside the blood holding chamber 531, the blood B1 moves radially outward and is held in a state of being gathered toward the inner wall 531a on the outside of the blood holding chamber 531 in the radial direction. Similarly, inside the isolation chamber 532, the specific gravity adjusting agent B2 moves radially outward and is held in a state of being gathered toward the inner wall 532a on the outside of the isolation chamber 532 in the radial direction.

Therefore, the blood B1 is kept in a state of having moved toward the inner wall 531a to which the first end 533a of the flow path 533 is connected. Also, the specific gravity adjusting agent B2 is kept in a state of having been gathered toward the inner wall 532a that is on the opposite side from the radially inner surface to which the flow path 533 is connected and is away from the second end 533b of the flow path 533.

That is, when centrifugal force is applied to the container 530, the liquid interface B2a of the specific gravity adjusting agent B2 held in the isolation chamber 532 is at a position that is away from the second end 533b of the connecting flow path 533 that connects the blood holding chamber 531 and the isolation chamber 532, as shown in Fig. 21A.

Consequently, the control unit 20 adjusts the rotation speed of the motor 16 to apply an appropriate amount of centrifugal force to the blood B1, so that the centrifugal force on the blood B1 is greater than the capillary action of the flow path 533, whereupon the blood moves a little at a time from the blood holding chamber 531 to the isolation chamber 532 via the flow path 533.

At this point, in the isolation chamber 532, the specific gravity adjusting agent B2 is kept at a position on the inner wall 532a side that is away from the second end 533b of the flow path 533, so the blood B1 that has moved to the isolation chamber 532 is stacked, without mixing with the specific gravity adjusting agent B2.

Here, the relation between the capillary action produced in the flow path 533 and the centrifugal force applied by rotation of the container 530 will be described using Figs. 7A and 7B in Embodiment 1 above.

With a hydrophobic capillary flow path (the flow path 533), when the rotation speed (centrifugal force) reaches a certain level, liquid feed through the flow path 533 is commenced.

This is because in the flow path 533, whose surface in contact with the blood B1 is hydrophobic, when no centrifugal force is being applied, capillary action (surface tension T) acts on the liquid, and as shown in Fig. 7A, a meniscus (curvature of the liquid surface formed by interaction with the surface of the container) that is convex to the left in the drawing is formed, and a force is generated that retains the liquid in the flow path 533.

In this state, when a centrifugal force greater than capillary action is applied to the container 530 along the direction of the flow path 533, as shown in Fig. 7B, the liquid (blood B1) in the flow path 533 moves radially outwards under this centrifugal force.

With the control unit 20 that controls the biological sample isolation device 10 in this embodiment, in the container 530 including the hydrophobic capillary flow path (flow path 533) described above, the rotation is controlled so that the proper amount of centrifugal force is applied to the container 530 in order for the blood B1 to be sent at the proper speed from the blood holding chamber 531 disposed on the inside in the radial direction, through the flow path 533, and to the isolation chamber 532.

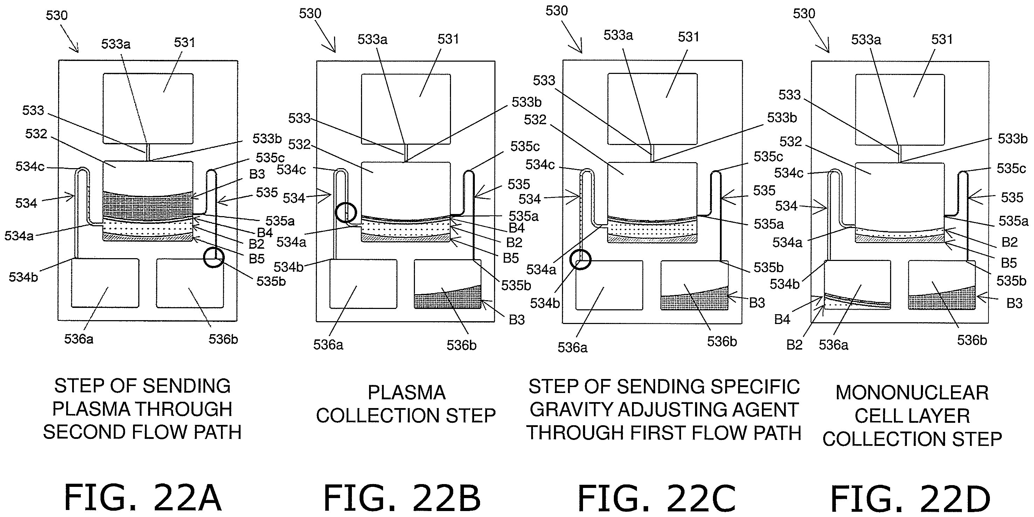

The collection chamber (collection unit) 536a is connected to the isolation chamber 532 by a hydrophilic flow path (first flow path 534) in which capillary action is produced, and is provided in order to isolate and collect a specific component (see the mononuclear cell layer B4 (Fig. 22D)) of the blood B1. As shown in Fig. 20B, the collection chamber 536a is disposed more to the outside in the radial direction than the rotational center O with respect to the isolation chamber 532 in a state of having been placed in the biological sample isolation device 10, and collects a specific component of the blood B1 (mononuclear cell layer B4).

The waste liquid holding chamber (waste liquid holding unit) 536b is connected to the isolation chamber 532 by a hydrophilic flow path (second flow path 535) in which capillary action is produced, and is provided in order to collect a specific component of the blood B1 (plasma B3). As shown in Fig. 20B, the waste liquid holding chamber 536b is disposed more to the outside in the radial direction than the rotational center O with respect to the isolation chamber 532 in a state of having been placed in the biological sample isolation device 10.

As shown in Fig. 20B, the hydrophilic first flow path 534 connects the isolation chamber 532 and the collection chamber 536a, and is a flow path in which capillary action is produced. When centrifugal force is applied to the first flow path 534 by rotation in the direction of the one-dot chain arrow in Fig. 20B, a specific component of the blood B 1 (mononuclear cell layer B4) and a part of the specific gravity adjusting agent B2 are moved by the siphon principle from the isolation chamber 532 to the collection chamber 536a.

The hydrophilic second flow path 535 connects the isolation chamber 532 and the waste liquid holding chamber 536b, and is a flow path in which is produced capillary action having a different magnitude from that of the first flow path 534. When centrifugal force is applied to the second flow path 535 by rotation in the direction of the one-dot chain arrow in Fig. 20B, the component that becomes the waste liquid (plasma B3) is moved by the siphon principle from the isolation chamber 532 to the waste liquid holding chamber 536b.

Here, the container 530 in this embodiment is configured so that there is a difference in the capillary action produced in the first flow path 534 and the second flow path 535, as described above.

More specifically, the first flow path 534 is formed to have a larger cross-sectional area (thickness) than the second flow path 535. Consequently, the capillary action produced in the second flow path 535 is larger than the capillary action produced in the first flow path 534.

Here, as shown in Fig. 20B, the same amount of centrifugal force is applied to the first flow path 534 and the second flow path 535, which are disposed at the same distance from the rotational center O in the radial direction. Since there is a difference in the capillary action produced in the first flow path 534 and the second flow path 535, the timing of the movement of the liquid through the first flow path 534 and the timing of the movement of the liquid through the second flow path 535 can be offset according to the change in centrifugal force. As a result, the isolation and collection of the specific component (mononuclear cell layer B4) from the first flow path 534 and the collection of waste liquid from the second flow path 535 can be controlled by varying the amount of centrifugal force through control of the rotation speed of the container 530.

Furthermore, as shown in Fig. 20B, the first flow path 534 has a first end 534a connected to the isolation chamber 532, a second end 534b connected to the collection chamber 536a, and a return part 534c formed between the first end 534a and the second end 534b.

As shown in Fig. 20B, the first end 534a is connected to the left side surface of the isolation chamber 532 that is substantially parallel to the radial direction. The first end 534a is connected at a position (first position) that is offset more to the outside in the radial direction than the first end 535a of the second flow path 535.

The second end 534b is connected to an end surface of the collection chamber 536a that is substantially perpendicular to the radial direction, as shown in Fig. 20B.

As shown in Fig. 20B, the return part 534c is a bent portion that is provided between the first end 534a and the second end 534b so as to connect the flow path (inward flow path) that exits the first end 534a and is formed facing radially inward at first, to a flow path (outward flow path) facing radially outward.

Similarly, as shown in Fig. 20B, the second flow path 535 has a first end 535a that is connected to the isolation chamber 532, a second end 535b that is connected to the collection chamber 536a, and a return part 535c formed between the first end 535a and the second end 535b.

As shown in Fig. 20B, the first end 535a is connected to the right side surface of the isolation chamber 532 that is substantially parallel to the radial direction. The first end 535a is connected at a position (second position) that is offset more to the inside in the radial direction than the first end 534a of the first flow path 534.

That is, the first flow path 534 is connected to the isolation chamber 532 at a position (first position) more to the outside than the second flow path 535 in the radial direction.

As shown in Fig. 20B, the second end 535b is connected to an end surface of the waste liquid holding chamber 536b that is substantially perpendicular to the radial direction.

As shown in Fig. 20B, the return part 535c is a bent portion that is provided between the first end 535a and the second end 535b so that the flow path, which is first formed facing inward in the radial direction after exiting the first end 535a, faces radially outward. As shown in Fig. 20B, the return part 535c is provided at a position that is substantially in left and right symmetry around the isolation chamber 532 with respect to the return part 534c on the first flow path 534 side.

Also, as shown in Fig. 20B, the return part 534c on the first flow path 534 side and the return part 535c on the second flow path 535 side are disposed at a position more to the inside in the radial direction than the liquid surface where the blood B 1 and the specific gravity adjusting agent B2 are stacked, in the step of isolating and collecting a specific component of the blood B1 by rotating the container 530.

Furthermore, with the container 530 of this embodiment, in a state in which centrifugal force is applied, the first position where the first flow path 534 is connected to the isolation chamber 532, and the second position where the second flow path 535 is connected to the isolation chamber 532 are disposed so as to sandwich the layer containing a specific component of the blood B1 (mononuclear cell layer B4) in the radial direction (see Fig. 21C).

Here, the relation between the capillary action produced in the first flow path 534 and the second flow path 535 and the centrifugal force applied by rotating the container 530 will be described through reference to Figs. 21B and 21C.

With a hydrophilic capillary flow path (the first flow path 534 and the second flow path 535), the flow of liquid through the first flow path 534 and the second flow path 535 is commenced when the rotation speed (centrifugal force) drops below a certain level.

This is because in a state in which no centrifugal force is being applied to the first flow path 534 and the second flow path 535, whose surfaces in contact with the blood B 1 are hydrophilic, capillary action (surface tension T) acts on the liquid, and as shown in Fig. 13A described in Embodiment 2 above, a concave state is formed on the left side in the figure, and in a state in which centrifugal force is applied, a force is generated that keeps the liquid in the first flow path 534 and the second flow path 535.

From this state, as shown in Fig. 13B, when the centrifugal force on the container 530 becomes less than the capillary action along direction of the first flow path 534 and the second flow path 535, the liquid (blood B1) in the flow path 534 and the second flow path 535 moves radially inward due to capillary action. This makes it possible to send the liquid (blood B1) in the first flow path 534 and the second flow path 535 while the rotation is stopped.

With the control unit 20 that controls the biological sample isolation device 10 in this embodiment, in this container 530 including the hydrophilic capillary flow paths (the first flow path 534 and the second flow path 535), rotation control is performed to change the amount of centrifugal force applied to the container 530 in order to send the specific component or waste liquid at the appropriate timing from the isolation chamber 532 disposed on the inside in the radial direction, through the first flow path 534 and the second flow path 535, to the collection chamber 536a and the waste liquid holding chamber 536b disposed on the outside in the radial direction.

More specifically, first, when the container 530 is rotated at a specific rotation speed by the biological sample isolation device 10, centrifugal force (large) is applied to the blood B 1 and the specific gravity adjusting agent B2 held in the isolation chamber 532 of the container 530, as shown in Fig. 21B. At this point, the blood B1 and the specific gravity adjusting agent B2 are pushed by centrifugal force toward the inner wall 532a on the outside of the isolation chamber 532 in the radial direction, and are held in a stacked state, without mixing with each other. At this point, the liquid level in the first flow path 534 and the second flow path 535 is kept at approximately the same height (in the radial direction).

Next, if the rotation of the container 530 is continued at the same speed (centrifugal force (large)) in the state shown in Fig. 21B, the blood cells are isolated from the state in which the blood B 1 and the specific gravity adjusting agent B2 are stacked, and the layers of plasma B3, mononuclear cell layer (specific component) B4, specific gravity adjusting agent B2, and red blood cells B5 are formed, in that order, from the inside in the radial direction.

Next, if the rotation speed of the container 530 is lowered to a specific level, the centrifugal force (large) applied to the liquid in the container 530 decreases to a centrifugal force (medium) as shown in Fig. 22A, and this sends the liquid in the second flow path 535 on the side where the capillary action is larger (the cross-sectional area is smaller). At this point, in the second flow path 535, as shown in Fig. 22A, waste liquid (plasma B3), which has the lowest specific gravity in the isolation chamber 532 is sent, which moves the liquid level to the position of connection to the waste liquid holding chamber 536b (second end 535b; see the circle in Fig. 22A).

The relation between capillary action and centrifugal force in the state of Fig. 22A is expressed by the following relational expression (1').

Capillary action in first flow path 534 < centrifugal force < capillary action in second flow path 535          (1')

That is, the rotation speed at which the container 530 is rotated in the state shown in Fig. 22A is set so as to satisfy the above relational expression (1'), according to the cross-sectional area of the first flow path 534 and the second flow path 535, etc.

Next, if the rotation speed of the container 530 is increased to a specific level again, as shown in Fig. 22B, the centrifugal force (medium) applied to the liquid in the container 530 increases to the centrifugal force (large). Consequently, the plasma B3 sent from the second end 535b of the second flow path 535 to the waste liquid holding chamber 536b is sent in a state in which the delivery speed has been raised by the increase in centrifugal force. Furthermore, since the inside of the second flow path 535 is filled with liquid, siphoning occurs, and all of the liquid up to the position where the first end 535a is connected in the isolation chamber 532 is sent.

At this point, in the second flow path 535, as shown in Fig. 22B, the plasma B3 up to a position more to the outside than the first end 535a in the radial direction is sent from inside the isolation chamber 532 to the waste liquid holding chamber 536b via the second flow path 535.

This causes almost all of the plasma B3 in the isolation chamber 532 to be sent to the waste liquid holding chamber 536b.

Also, at this point, since the plasma B3 is sent to the waste liquid holding chamber 536b and the liquid level in the isolation chamber 532 moves radially outward, the liquid level held in the first flow path 534 also moves to about the same position (see the circle in Fig. 22B).

Next, if the rotation speed of the container 530 is lowered to a specific level again, the centrifugal force (large) applied to the liquid in the container 530 decreases to the centrifugal force (small). At this point, in the first flow path 534, the specific gravity adjusting agent B2 and the mononuclear cell layer B4 remaining in the isolation chamber 532 are sent from the first end 534a of the first flow path 534 to the second end 534b (see the circle in Fig. 22C).

At this point, the centrifugal force (small) applied to the liquid in the isolation chamber 532 is even smaller than the centrifugal force (medium) when sending the waste liquid shown in Fig. 21C. The relation between capillary action and centrifugal force in the state at this point is expressed by the following relational expression (2').

$$\text{Capillary action in first flow path } 534 > \text{centrifugal force} \ \dots \ (2')$$

That is, capillary action becomes dominant when the rotation of the container 530 is controlled so that the centrifugal force (small) applied to the liquid in the isolation chamber 532 is smaller than the capillary action produced in the first flow path 534. Consequently, the liquid is sent through the first flow path 534, and the liquid level moves to the position of the connection portion (second end 534b) with the collection chamber 536a.

Next, if the rotation speed of the container 530 is increased to a specific level again, the centrifugal force (small) applied to the liquid in the container 530 increases to the centrifugal force (large) as shown in Fig. 22D, which causes the mononuclear cell layer B4 sent from the second end 534b into the collection chamber 536a while increasing the liquid delivery speed through an increase in centrifugal force.

At this point, in the first flow path 534, as shown in Fig. 22D, the mononuclear cell layer B4, the specific gravity adjusting agent B2, etc., up to a position more to the outside than the first end 534a in the radial direction are sent from the isolation chamber 532 to the collection chamber 536a via the first flow path 534.

The relation between capillary action and centrifugal force in the state of Fig. 22D is expressed by the following relational expression (3').

$$\text{Capillary action of first flow path } 534 > \text{centrifugal force} \ \dots \ (3)$$

Consequently, the liquid (mononuclear cell layer B4, etc.) held in the first flow path 534 moves radially outward under centrifugal force. Furthermore, since the inside of the first flow path 534 is filled with liquid, siphoning occurs, and all the liquid up to the position where the first end 534a is connected in the isolation chamber 532 is sent.

Biological Sample Isolation Control Method

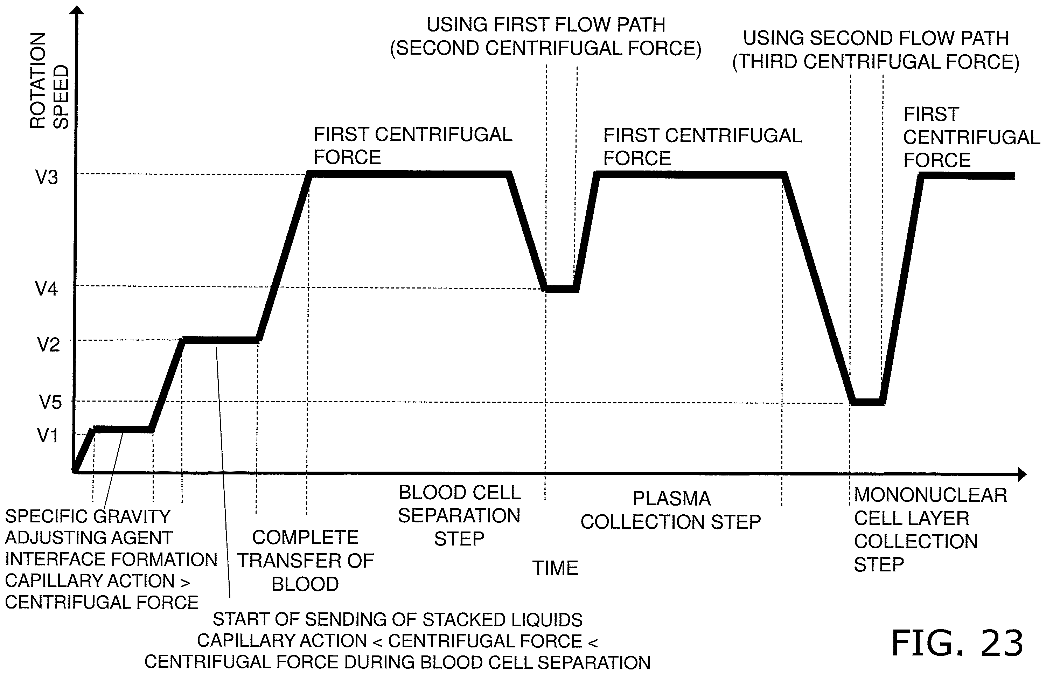

With the biological isolation control method of this embodiment, as shown in Fig. 23, the control unit 20 controls the motor 16 so as to vary the centrifugal force applied to the isolated components of the blood B1 by setting the rotation speed at which the container 530 is rotated in five stages of V1, V2, V3, V4, and V5 and by changing the rotation speed.

That is, in a state in which the container 530 has been placed in the container holder 12a of the pullout section 12 of the biological sample isolation device 10, the control unit 20 rotates the motor 16 and increases the rotation speed to V1 as shown in Fig. 23.

At this point, in the container 530, centrifugal force forms a liquid interface B2a at a position where the specific gravity adjusting agent B2 in the isolation chamber 532 is away from the second end 533b of the flow path 533 (formation of interface of the specific gravity adjusting agent B2). At the same time, the blood B1 in the blood holding chamber 531 is gathered toward the inner wall 531a on the outside in the radial direction by centrifugal force, and is kept within the flow path 533 from the first end 533a of the flow path 533 by capillary action.

As shown in Fig. 23, the rotation speed V1 is set so that the capillary action acting on the blood B1 in the flow path 533 and the centrifugal force generated by rotation will satisfy the following relational expression (3).

$$\text{Capillary action} > \text{centrifugal force} \quad \dots (3)$$

That is, the control unit 20 has a first control that controls the rotation speed of the container 530 so that a centrifugal force smaller than the capillary action generated in the flow path 533 is applied to gather the blood B1 and the specific gravity adjusting agent B2 in a state of being offset toward the radially outer surface of the blood holding chamber 531 and the isolation chamber 532, and a second control that controls the rotation speed of the container 530 so that a centrifugal force greater than the capillary action generated in the flow path 533 is applied to send the blood B1 through the flow path 533.

Then, once the above state stabilizes, the control unit 20 increases the rotation speed of the motor 16 to V2.

At this point, in the container 530, as shown in Fig. 23, a centrifugal force that is greater than that at the rotation speed V1 starts sending the blood B1 in the blood holding chamber 531 a little at a time through the flow path 533 to the isolation chamber 532, while the liquid interface B2a is still formed at a position where the specific gravity adjusting agent B2 in the isolation chamber 532 is away from the second end 533b of the flow path 533 (start of stacked liquid feed).

As shown in Fig. 23, the rotation speed V2 is set such that the capillary action acting on the blood B1 in the flow path 533, the centrifugal force generated by rotation, and the centrifugal force during blood cell isolation satisfy the following relation.

Capillary action < centrifugal force < centrifugal force during blood cell isolation          (4)

Then, the blood B 1 is sent to through the flow path 533 to the isolation chamber 532 by the centrifugal force applied at the rotation speed V2, and once almost all of the blood B 1 has been sent out, the control unit 20 further increases the rotation speed of the motor 16 to V3.

At this point, in the container 530, as shown in Fig. 23, the blood B1 in the blood holding chamber 531 is completely sent through the flow path 533 to the isolation chamber 532 by a centrifugal force larger than that at the rotation speed V2 before the rotation speed changes from V2 to V3 (complete transfer of blood B1).

The rotation speed V3 is set so that a third centrifugal force used for blood cell isolation is applied, as shown in Fig. 23.

When the rotation speed V3 is reached, a first centrifugal force is applied to the liquid (blood B1, etc.) in the container 530. Then, after a specific amount of time has elapsed in this state, blood cell isolation proceeds from a state in which the blood B1 and the specific gravity adjusting agent B2 in the isolation chamber 532 are stacked (blood cell isolation step).

Next, when a specific amount of time has elapsed, the control unit 20 determines that blood cell isolation has been completed, and reduces the rotation speed of the motor 16 from V3 to V4, as shown in Fig. 23.

At this point, when the rotation speed reaches V4, a second centrifugal force is applied to the liquid in the container 530. The relation between capillary action and the second centrifugal force is expressed by the above-mentioned relational expression (1').

Capillary action in first flow path 534 < centrifugal force < capillary action in second flow path 535          (1')

Consequently, the waste liquid (plasma B3) with the lowest specific gravity is sent out from inside the isolation chamber 532, and the liquid level moves to the position of the connection portion (second end 535b) with the waste liquid holding chamber 536b.

Next, as shown in Fig. 23, when the control unit 20 increases the rotation speed of the container 530 from V4 to V3 again, the centrifugal force applied to the liquid in the container 530 increases from the second centrifugal force to the first centrifugal force.

Consequently, the plasma B3 sent from the second end 535b of the second flow path 535 is sent while increasing the delivery speed due to the increase in centrifugal force. Furthermore, since the inside of the second flow path 535 is filled with liquid, siphoning occurs, and all of the liquid up to the position where the first end 435a is connected in the isolation chamber 431 is sent (plasma collection step).

Next, as shown in Fig. 23, when the control unit 20 again lowers the rotation speed of the container 530 from V3 to V5, the centrifugal force applied to the liquid in the container 530 decreases from the first centrifugal force to the third centrifugal force. Consequently, the specific gravity adjusting agent B2 and the mononuclear cell layer B4 remaining in the isolation chamber 532 are sent from the first end 534a of the first flow path 534 to the second end 534b.

At this point, the relation between the third centrifugal force applied to the liquid in the isolation chamber 532 and capillary action is expressed by the following relational expression (2'), as mentioned above.

$$\text{Capillary action of first flow path } 534 > \text{centrifugal force } \ldots (2')$$

That is, capillary action becomes dominant when the rotation of the container 530 is controlled so that the third centrifugal force applied to the liquid in the isolation chamber 532 is smaller than the capillary action produced in the first flow path 534. Consequently, the liquid is sent through the first flow path 534, and the liquid level moves to the position of the connection portion (second end 534b) with the collection chamber 536a.

Next, as shown in Fig. 23, when the control unit 20 again increases the rotation speed of the container 530 from V5 to V1, the third centrifugal force applied to the liquid in the container 530 increases to the first centrifugal force. Consequently, the mononuclear cell layer B4 sent from the second end 534b is sent from the second end 534b into the collection chamber 536a while increasing the liquid delivery speed by an increase in centrifugal force (mononuclear cell layer collection step).

Addendum 1

The biological sample isolation control device according to the first disclosure is:
a biological sample isolation control device that controls a rotation device so that a container in which a biological sample and a reagent have been placed in isolate spaces is rotated by the rotation device so as to stack the biological sample and the reagent, wherein:

the container has a first holding unit that holds the biological sample, a second holding unit that holds the reagent, and a hydrophobic flow path that connects the first holding unit and the second holding unit and in which capillary action acts,
the rotation device has a container holder that holds the container such that the first holding unit and the second holding unit are disposed at different distances in a radial direction from the rotational center, and a rotation drive unit that rotates the container around the rotational center, and
a control unit is provided that controls the rotation drive unit so that the biological sample or the reagent is moved between the first holding unit and the second holding unit via the flow path, and the biological sample and the reagent are stacked.

Addendum 2

The biological sample isolation control device according to the second disclosure is the biological sample isolation control device according to the first disclosure, wherein:
the flow path is disposed along the radial direction in a state in which the container has been placed in the container holder.

Addendum 3

The biological sample isolation control device according to the third disclosure is the biological sample isolation control device according to the first or second disclosure, wherein:
the control unit controls the rotation drive unit so that a centrifugal force greater than the capillary action produced in the flow path is applied by rotation by the rotation device.

Addendum 4

The biological sample isolation control device according to the fourth disclosure is the biological sample isolation control device according to the third disclosure, wherein:
the control unit has a first control that controls the rotation speed of the container so that a centrifugal force smaller than the capillary action generated in the flow path is applied to gather the blood and the specific gravity adjusting agent in a state of being offset toward the radially outer surface of the first holding unit and the second holding unit, and a second control that controls the rotation speed of the container so that a centrifugal force greater than the capillary action generated in the flow path is applied to send the blood through the flow path.

Addendum 5

The biological sample isolation control device according to the fifth disclosure is the biological sample isolation control device according to the fourth disclosure, wherein:
after the second control, the control unit rotates the container at a rotation speed higher in than the second control in a state in which the biological sample and the reagent are stacked, so that a specific component contained in the biological sample is isolated.

Addendum 6

The biological sample isolation control device according to the sixth disclosure is the biological sample isolation control device according to the fourth or fifth disclosure, wherein:
in the second control, the control unit controls the movement speed of the biological sample or the reagent by adjusting the rotation speed of the rotation drive unit.

Addendum 7

The biological sample isolation control device according to the seventh disclosure is the biological sample isolation control device according to any of the first to sixth disclosures, wherein:
in a state in which the container has been placed in the container holder, the first holding unit is disposed closer to the rotational center in the radial direction than the second holder.

Addendum 8

The biological sample isolation control device according to the eighth disclosure is the biological sample isolation control device according to the seventh disclosure, wherein:
when a centrifugal force greater than the capillary action generated in the flow path is applied by rotation by the rotation device, the biological sample moves from the first holding unit to the second holding unit via the flow path.

Addendum 9

The biological sample isolation control device according to the ninth disclosure is the biological sample isolation control device according to the seventh or eighth disclosure, wherein:
in a state in which centrifugal force is applied to the container by the rotation device, the connection portion between the flow path and the second holding unit is disposed closer to the rotational center than the liquid interface of the reagent.

Addendum 10

The biological sample isolation control device according to the tenth disclosure is the biological sample isolation control device according to any of the seventh to ninth disclosures, wherein:
when a centrifugal force is applied to the container by the rotation device, in the second holding unit disposed on the radially outer side, the reagent is kept in a state of being gathered to the radially outer surface.

Addendum 11

The biological sample isolation control device according to the eleventh disclosure is the biological sample isolation control device according to any of the first to tenth disclosures, wherein:

    in a state in which the container has been placed in the container holder, the flow path has a first end that is connected to the first holding unit and a second end that is connected to the second holding unit,
    the first end is connected to a radially outer surface of the first holding unit, and
    the second end is connected to a radially inner surface of the second holding unit.

Addendum 12

The biological sample isolation control device according to the twelfth disclosure is the biological sample isolation control device according to any of the first to tenth disclosures, wherein:

in a state in which the container has been placed in the container holder, the flow path has a first end that is connected to the first holding unit and a second end that is connected to the second holding unit,
the first end is connected to a radially outer surface of the first holding unit, and
the second end is connected to a surface of the second holding unit extending in the radial direction, and more to the inside in the radial direction than the liquid surface of the reagent in a state in which centrifugal force is applied.

Addendum 13

The biological sample isolation control device according to the thirteenth disclosure is the biological sample isolation control device according to any of the first to tenth disclosures, wherein:

the first holding unit is disposed more to the inside in the radial direction than the second holding unit, and has a first wall surface that is disposed radially outside and is connected to the flow path, and
the first wall surface has a concave surface where the connection portion with the flow path is disposed at the outermost side in the radial direction.

Addendum 14

The biological sample isolation control device according to the fourteenth disclosure is the biological sample isolation control device according to any of the first to tenth disclosures, wherein:

the second holding unit is disposed more to the inside in the radial direction than the first holding unit, and
the flow path is connected to the second holding unit on a radially outer surface, and connected to the first holding unit on a radially outer surface.

Addendum 15

The biological sample isolation control method according to the fifteenth disclosure is:
a biological sample isolation control method using the biological sample isolation control device according to any of the first to fourteenth disclosures, comprising:

a first step of controlling the rotational drive unit to a rotation speed such that the biological sample held in the first holding unit and the reagent held in the second holding unit are gathered toward a surface on the outside in the radial direction in the first holding unit and the second holding unit, respectively;
a second step of controlling the rotational drive unit to a rotation speed such that the biological sample or the reagent moves through the flow path from the first holding unit to the second holding unit or from the second holding unit to the first holding unit; and
a third step of controlling the rotational drive to a rotation speed such that a specific component is isolated from the biological sample in a state in which the biological sample and the reagent are stacked due to the movement of the biological sample or the reagent in the second step.

Addendum 16

The biological sample isolation control program according to the sixteenth disclosure causes a computer to execute the biological sample isolation control method according to the fifteenth disclosure.

INDUSTRIAL APPLICABILITY

The biological sample isolation container of the present invention exhibits the effect that variance in collection accuracy is less likely to occur when taking out and collecting a specific component of a biological sample isolated into components from the container, and as such is widely applicable to various kinds of container used in the isolation of biological samples.

REFERENCE SIGNS LIST

10 biological sample isolation device (rotation device)
11 housing unit
11a, 11b opening
12 pullout section
12a container holder
13 base member
13a opening
14 display unit
15 power button
16 motor (rotation drive unit)
17 board unit
20 control unit (biological sample isolation control device, control unit)
30 container
31 first holding unit
31a inner wall
32 second holding unit
32a inner wall
33 flow path
33a first end
33b second end
130 container
133 flow path
133a first end
133b second end
230 container
231 first holding unit
231a inner wall
232 second holding unit
232a inner wall
233 flow path
233a first end
233b second end
330 container
331 first holding unit
331a inner wall
332 second holding unit
332a inner wall
333 flow path
333a first end
333b second end
430 container (biological sample isolation container)
431 isolation chamber (isolation unit)
431a inner wall
432 collection chamber (collection unit)
433 waste liquid holding chamber (waste liquid holding unit)
434 first flow path
434a first end
434b second end
434c return part
435 second flow path
435a first end
435b second end
435c return part
530 container (biological sample isolation container)
531 blood holding chamber (blood holding unit)
531a inner wall
532 isolation chamber (isolation unit)
532a inner wall

533 flow path
533a first end
533b second end
534 first flow path
534a first end
534b second end
534c return part
535 second flow path
535a first end
535b second end
535c return part
536a collection chamber (collection unit)
536b waste liquid holding chamber (waste liquid holding unit)
537 cover
537aa injection hole
537ab injection hole
537b air hole
B1 blood (biological sample)
B2 specific gravity adjusting agent (reagent)
B2a liquid interface
B3 plasma
B4 mononuclear cell layer (specific component)
B5 red blood cells
O center of rotation

## Claims

1. A biological sample isolation container for recovering a specific component contained in a biological sample isolated into components, by rotating in a state of being placed in a rotation device, the biological sample isolation container comprising:

an isolation unit configured to store a biological sample and a reagent;
a collection unit that is disposed on an outside in a radial direction from a rotational center with respect to the isolation unit in a state of being set in the rotation device, and configured to collect a specific component of the biological sample;
a waste liquid holding unit that is disposed on the outside in the radial direction from the rotational center with respect to the isolation unit in a state of being set in the rotation device, and configured to hold waste liquid of the reagent and the biological sample excluding the specific component;
a hydrophilic first flow path configured to connect the isolation unit and the collection unit and cause capillary action to move the specific component from the isolation unit to the collection unit by a siphon principle when centrifugal force is applied by a rotation; and
a hydrophilic second flow path configured to connect the isolation unit and the waste liquid holding unit cause a capillary action whose magnitude is different from that of the first flow path to move the waste liquid from the isolation unit to the waste liquid holding unit by the siphon principle when centrifugal force is applied by the rotation,
wherein the first flow path is connected to a first position on a first side surface of the isolation unit,
the second flow path is connected at a second position on a second side surface that is on an opposite side from the first side surface in the isolation unit, and
the first position and the second position are provided at positions offset in the radial direction in a state of being set in the rotation device.

2. The biological sample isolation container according to claim 1,
wherein the capillary action generated in the second flow path is greater than the capillary action generated in the first flow path.

3. The biological sample isolation container according to claim 2,
wherein the second flow path has a smaller cross-sectional area than the first flow path.

**4.** The biological sample isolation container according to any of claims 1 to 3,
wherein the first position and the second position are disposed so as to sandwich a layer containing the specific component in the radial direction when the centrifugal force is applied.

**5.** The biological sample isolation container according to any of claims 1 to 4,
wherein, in a state of being set in the rotation device, the first flow path and the second flow path include an inward flow path that faces inward in the radial direction from a portion connected to the isolation unit, a return part that is bent back so as to face outward in the radial direction from the inward flow path, and an outward flow path that is connected to the collection unit and the waste liquid holding unit and faces outward in the radial direction from the return part.

**6.** The biological sample isolation container according to claim 5,
wherein the return part is provided at a position more to an inside in the radial direction than a liquid level of the biological sample and the reagent in the isolation unit in a state in which the centrifugal force is applied.

**7.** The biological sample isolation container according to claim 1, further comprising:

a biological sample holding unit that is disposed on an inside in the radial direction centered on a rotational center with respect to the isolation unit, and configured to hold a biological sample; and
a capillary flow path configured to connect the biological sample holding unit and the isolation unit.

**8.** A biological sample isolation control device that controls the rotation of a rotation device in which the biological sample isolation container according to any of claims 1 to 7 is placed,
the biological sample isolation control device comprising a rotation control unit configured to control a drive unit of the rotation device.

**9.** The biological sample isolation control device according to claim 8,
wherein the rotation control unit has:

a first mode in which the drive unit of the rotation device is rotated at a first speed so that the biological sample and the reagent are held in a state of being offset toward a radially outer surface of the isolation unit; and
a second mode in which, when the biological sample is isolated in the isolation unit in the first mode, the drive unit of the rotation device is rotated at a second speed that is lower than the first speed, thereby sending the waste liquid through the second flow path to the waste liquid holding unit.

**10.** The biological sample isolation control device according to claim 9,
further having a third mode in which, when the waste liquid is sent to the waste liquid holding unit in the second mode, the rotation control unit causes the drive unit of the rotation device to rotate at a third speed that is higher than the second speed, thereby sending the waste liquid from inside the isolation unit to the waste liquid holding unit until there is no more waste liquid.

**11.** The biological sample isolation control device according to claim 10,
further having a fourth mode in which, when the sending of the waste liquid to the waste liquid holding unit is completed in the third mode, the rotation control unit causes the drive unit of the rotation device to rotate at a fourth speed that is lower than the third speed, thereby sending the specific component through the first flow path from the isolation unit to the collection unit.

**12.** The biological sample isolation control device according to claim 11,
further having a fifth mode in which, when the specific component is sent to the collection unit in the fourth mode, the rotation control unit causes the drive unit of the rotation device to rotate at a fifth speed that is higher than the fourth speed, thereby sending the specific component from within the isolation unit until there is no more of the specific component.

**13.** The biological sample isolation control device according to claim 9,
wherein, as a preliminary stage of the first mode, the rotation control unit rotates the biological sample isolation unit such that the biological sample is sent from the biological sample holding unit, which is disposed inside the isolation unit in the radial direction centered on the rotational center and which holds the biological sample, through a capillary flow path connecting the sample holding unit and the isolation unit, to the isolation unit, and the reagent and the biological sample are stacked in the isolation unit.

14. A biological sample isolation control method for controlling the rotation of a rotation device in which the biological sample isolation container according to any of claims 1 to 7 is placed, the method comprising:

a first step of rotating a drive unit of the rotation device at a first speed so that the biological sample and the reagent are held in a state of being offset toward a radially outer surface of the isolation unit; and
a second step of rotating the drive unit of the rotation device at a second speed that is lower than the first speed when the biological sample is isolated in the isolation unit in the first step, thereby sending the waste liquid through the second flow path to the waste liquid holding unit.

15. The biological sample isolation control method according to claim 14,
further comprising a third step of rotating the drive unit of the rotation device at a third speed that is higher than the second speed when the waste liquid is sent to the waste liquid holding unit in the second step, thereby sending the waste liquid from inside the isolation unit until there is no more of the waste liquid.

16. The biological sample isolation control method according to claim 15,
further comprising a fourth step of rotating the drive unit of the rotation device at a fourth speed that is lower than the third speed when the sending of the waste liquid to the waste liquid holding unit is completed in the third step, thereby sending the specific component through the first flow path from the isolation unit to the collection unit.

17. The biological sample isolation control method according to claim 16,
further comprising a fifth step of rotating the drive unit of the rotation device at a fifth speed that is higher than the fourth speed when the specific component is send to the collection unit in the fourth step, thereby sending the specific component from inside the isolation unit to the collection unit until there is no more of the specific component.

18. The biological sample isolation control method according to claim 14,
further comprising, as a preliminary stage of the first step, a step of rotating the biological sample isolation container such that the biological sample is sent from the biological sample holding unit, which is disposed inside the isolation unit in the radial direction centered on the rotational center and which holds the biological sample, through a capillary flow path connecting the sample holding unit and the isolation unit, to the isolation unit, and the reagent and the biological sample are stacked in the isolation unit.

19. A biological sample isolation control program,
which causes a computer to execute the biological sample isolation control method according to any of claims 14 to 18.

EP 4 455 674 A1

FIG. 1

FIG. 2

FIG. 3

EP 4 455 674 A1

FIG. 4

FIG. 5

FIG. 6

HYDROPHOBIC
FLOW PATH

SURFACE
TENSION T

CAPILLARY
ACTION

SURFACE
TENSION T

METHOD FOR SENDING
LIQUID DURING ROTATION

## FIG. 7A

ROTATIONAL
AXIS

SURFACE
TENSION T

CAPILLARY
ACTION

CENTRIFUGAL
FORCE

SURFACE
TENSION T    HYDROPHOBIC

LIQUID SENDING
DURING ROTATION

## FIG. 7B

FIG. 8

EP 4 455 674 A1

```
                          ┌─────────┐
                          │  START  │
                          └────┬────┘
                               ▼
              ┌──────────────────────────────────┐
              │ PLACE CONTAINER IN BIOLOGICAL     │── S11
              │ SAMPLE SEPARATION DEVICE          │
              └───────────────┬──────────────────┘
                              ▼
              ┌──────────────────────────────────┐
              │    START ROTATION OF MOTOR        │── S12
              └───────────────┬──────────────────┘
                              ▼
                  ╱─────────────────────╲    NO
                 ╱  HAS ROTATION SPEED    ╲ ───────┐
                 ╲   REACHED V1?          ╱ S13     │
                  ╲─────────────────────╱           │
                        YES ▼ ◄──────────────────── │
                  ╱─────────────────────────╲       │
                 ╱    HAS LIQUID              ╲  NO  │
                ╱ INTERFACE OF SPECIFIC GRAVITY╲────┤
                ╲ ADJUSTING AGENT FORMED IN    ╱ S14 │
                 ╲    SECOND HOLDING          ╱      │
                  ╲     UNIT?                ╱       │
                   ╲─────────────────────╱          │
                        YES ▼                       │
              ┌──────────────────────────────┐ S15  │
              │  RAISE MOTOR ROTATION SPEED   │      │
              └───────────────┬──────────────┘      │
                              ▼                      │
                  ╱─────────────────────╲    NO      │
                 ╱      HAS               ╲──────────┤
                ╱  ROTATION SPEED          ╲         │
                ╲   REACHED V2?            ╱ S16      │
                 ╲─────────────────────╱             │
                        YES ▼           S17           │
              ┌──────────────────────────────┐        │
              │ BLOOD MOVES THROUGH FLOW PATH │        │
              │ TO SECOND HOLDING UNIT, AND   │        │
              │ IS STACKED WITH SPECIFIC      │        │
              │ GRAVITY ADJUSTING AGENT       │        │
              └───────────────┬──────────────┘
```

```
                  ╱─────────────────────╲    NO
                 ╱        HAS             ╲ ───────┐
                ╱  SPECIFIC AMOUNT OF      ╲        │
                ╲   TIME ELAPSED?          ╱ S18    │
                 ╲─────────────────────╱           │
                        YES ▼                       │
              ┌──────────────────────────────┐ S19  │
              │  RAISE MOTOR ROTATION SPEED   │      │
              └───────────────┬──────────────┘      │
                              ▼                      │
              ┌──────────────────────────────┐      │
              │ MOVEMENT OF BLOOD THROUGH     │ S20  │
              │ FLOW PATH TO SECOND HOLDING   │      │
              │ UNIT COMPLETE                 │      │
              └───────────────┬──────────────┘      │
                              ▼                      │
                  ╱─────────────────────╲    NO      │
                 ╱  HAS ROTATION SPEED    ╲──────────┤
                 ╲   REACHED V3?          ╱ S21       │
                  ╲─────────────────────╱            │
                        YES ▼ ◄──────────────────────┘
                  ╱─────────────────────╲    NO
                 ╱    HAS SPECIFIC        ╲ ─────────
                ╱  AMOUNT OF TIME          ╲
                ╲   ELAPSED?               ╱ S22
                 ╲─────────────────────╱
                        YES ▼
              ┌──────────────────────────────┐
              │    STOP MOTOR ROTATION        │── S23
              └───────────────┬──────────────┘
                              ▼
                          ┌─────────┐
                          │   END   │
                          └─────────┘
```

FIG. 9

FIG. 10A  FIG. 10B  FIG. 10C

EP 4 455 674 A1

FIG. 11

FIG. 12

HYDROPHOBIC
FLOW PATH

SURFACE
TENSION T

CAPILLARY
ACTION

SURFACE
TENSION T

METHOD FOR SENDING LIQUID
AFTER ROTATION IS STOPPED

# FIG. 13A

ROTATIONAL
AXIS

SURFACE
TENSION T

CAPILLARY
ACTION

SURFACE
TENSION T

CENTRIFUGAL
FORCE

HYDROPHOBIC

LIQUID SENDING
AFTER ROTATION
IS STOPPED

# FIG. 13B

FIG. 14A

FIG. 14B

FIG. 14C

SENDING OF PLASMA TO
WASTE LIQUID CHAMBER

CENTRIFUGAL
FORCE (LARGE)

FIG. 15A

SENDING OF MONONUCLEAR
CELL LAYER THROUGH
FLOW PATH (LEFT SIDE)

CENTRIFUGAL
FORCE (SMALL)

FIG. 15B

SENDING OF
MONONUCLEAR CELL LAYER
TO COLLECTION CHAMBER

CENTRIFUGAL
FORCE (LARGE)

FIG. 15C

FIG. 16

EP 4 455 674 A1

```
                    ( START )
                        │
                        ▼
        ┌─────────────────────────────────┐
        │ PLACE CONTAINER IN BIOLOGICAL   │── S31
        │ SAMPLE SEPARATION DEVICE        │
        └─────────────────────────────────┘
                        │
                        ▼
        ┌─────────────────────────────────┐
        │    START ROTATION OF MOTOR      │── S32
        └─────────────────────────────────┘
                        │
                        ▼
              ◇ HAS ROTATION SPEED        NO
                REACHED V1?  ── S33
                        │ YES
                        ▼
              ◇ HAS SPECIFIC LENGTH OF    NO
                TIME FOR COMPLETION OF
                BLOOD CELL SEPARATION
                ELAPSED?  ── S34
                        │ YES
                        ▼
        ┌─────────────────────────────────┐
        │   LOWER MOTOR ROTATION SPEED    │── S35
        └─────────────────────────────────┘
                        │
                        ▼
              ◇ HAS ROTATION SPEED        NO
                REACHED V2?  ── S36    S37
                        │ YES
                        ▼
        ┌─────────────────────────────────┐
        │ PLASMA IS SENT THROUGH SECOND   │
        │ FLOW PATH TO WASTE LIQUID       │
        │ HOLDING CHAMBER                 │
        └─────────────────────────────────┘
```

```
        ┌─────────────────────────────────┐
        │    RAISE MOTOR ROTATION SPEED   │── S38
        └─────────────────────────────────┘
                        │
                        ▼
              ◇ HAS SPECIFIC AMOUNT OF    NO
                TIME ELAPSED?  ── S39
                        │ YES
                        ▼
        ┌─────────────────────────────────┐
        │   LOWER MOTOR ROTATION SPEED    │── S40
        └─────────────────────────────────┘
                        │
                        ▼
              ◇ HAS ROTATION SPEED        NO
                REACHED V3?  ── S41
                        │ YES
                        ▼
        ┌─────────────────────────────────┐
        │ SEND MONONUCLEAR CELL LAYER     │
        │ THROUGH FIRST FLOW PATH TO      │── S42
        │ COLLECTION CHAMBER              │
        └─────────────────────────────────┘
                        │
                        ▼
        ┌─────────────────────────────────┐
        │    RAISE MOTOR ROTATION SPEED   │── S43
        └─────────────────────────────────┘
                        │
                        ▼
              ◇ HAS SPECIFIC AMOUNT OF    NO
                TIME ELAPSED?  ── S44
                        │ YES
                        ▼
        ┌─────────────────────────────────┐
        │      STOP MOTOR ROTATION        │── S45
        └─────────────────────────────────┘
                        │
                        ▼
                     ( END )
```

FIG. 17

FIG. 18

EP 4 455 674 A1

FIG. 19B

FIG. 19A

FIG. 20B

FIG. 20A

FIG. 21A

SPECIFIC GRAVITY ADJUSTING AGENT INTERFACE FORMATION

FIG. 21B

STACKED SENDING OF LIQUID

FIG. 21C

BLOOD CELL SEPARATION STEP

STEP OF SENDING
PLASMA THROUGH
SECOND FLOW PATH

FIG. 22A

PLASMA
COLLECTION STEP

FIG. 22B

STEP OF SENDING SPECIFIC
GRAVITY ADJUSTING AGENT
THROUGH FIRST FLOW PATH

FIG. 22C

MONONUCLEAR
CELL LAYER
COLLECTION STEP

FIG. 22D

EP 4 455 674 A1

50

FIG. 23

## INTERNATIONAL SEARCH REPORT

| International application No. |
|---|
| **PCT/JP2022/044876** |

**A. CLASSIFICATION OF SUBJECT MATTER**

*G01N 35/00*(2006.01)i; *B04B 5/04*(2006.01)i; *B04B 9/10*(2006.01)i; *B04B 13/00*(2006.01)i; *G01N 1/10*(2006.01)i; *G01N 33/48*(2006.01)i; *G01N 37/00*(2006.01)i

FI: G01N35/00 D; B04B5/04; B04B9/10; B04B13/00; G01N33/48 C; G01N37/00 101; G01N1/10 H

According to International Patent Classification (IPC) or to both national classification and IPC

**B. FIELDS SEARCHED**

Minimum documentation searched (classification system followed by classification symbols)

G01N35/00; B04B5/04; B04B9/10; B04B13/00; G01N1/10; G01N33/48; G01N37/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2007-163342 A (MATSUSHITA ELECTRIC IND CO LTD) 28 June 2007 (2007-06-28) paragraphs [0028]-[0036], fig. 2 | 1-12, 14-17, 19 |
| Y | JP 2010-151683 A (SHARP CORP) 08 July 2010 (2010-07-08) paragraph [0044], abstract | 1-19 |
| Y | JP 2007-232674 A (MATSUSHITA ELECTRIC IND CO LTD) 13 September 2007 (2007-09-13) paragraphs [0028]-[0032], fig. 7-10 | 1-19 |
| Y | JP 2008-542743 A (SPINX, INC.) 27 November 2008 (2008-11-27) paragraph [0132] | 1-19 |
| A | WO 2009/044552 A1 (PANASONIC CORPORATION) 09 April 2009 (2009-04-09) entire text, all drawings | 1-19 |
| A | US 2009/0246082 A1 (SAIKI, Hiroshi) 01 October 2009 (2009-10-01) paragraphs [0136]-[0157], fig. 12-14 | 1-19 |

☑ Further documents are listed in the continuation of Box C.      ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **08 February 2023** | **21 February 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

<div align="center">

**INTERNATIONAL SEARCH REPORT**

</div>

| International application No. |
|---|
| **PCT/JP2022/044876** |

**C.    DOCUMENTS CONSIDERED TO BE RELEVANT**

| Category* | Citation of document, with indication, where appropriate, of the relevant passages | Relevant to claim No. |
|---|---|---|
| A | US 2010/0297659 A1 (YOO, Jae-Chern) 25 November 2010 (2010-11-25) entire text, all drawings | 1-19 |

Form PCT/ISA/210 (second sheet) (January 2015)

INTERNATIONAL SEARCH REPORT
Information on patent family members

International application No.

**PCT/JP2022/044876**

| Patent document cited in search report | | | Publication date (day/month/year) | Patent family member(s) | | | Publication date (day/month/year) |
|---|---|---|---|---|---|---|---|
| JP | 2007-163342 | A | 28 June 2007 | (Family: none) | | | |
| JP | 2010-151683 | A | 08 July 2010 | US | 2010/0170789 | A1 | |
| | | | | paragraph [0066], abstract | | | |
| JP | 2007-232674 | A | 13 September 2007 | (Family: none) | | | |
| JP | 2008-542743 | A | 27 November 2008 | US | 2009/0039000 | A1 | |
| | | | | paragraph [0166] | | | |
| | | | | WO | 2007/057788 | A2 | |
| | | | | CN | 101291736 | A | |
| WO | 2009/044552 | A1 | 09 April 2009 | US | 2010/0221741 | A1 | |
| | | | | entire text, all drawings | | | |
| | | | | US | 2013/0164763 | A1 | |
| | | | | EP | 2209008 | A1 | |
| | | | | EP | 3141901 | A1 | |
| | | | | EP | 3447494 | A1 | |
| | | | | CN | 101796420 | A | |
| | | | | CN | 102879558 | A | |
| | | | | CN | 103424543 | A | |
| US | 2009/0246082 | A1 | 01 October 2009 | (Family: none) | | | |
| US | 2010/0297659 | A1 | 25 November 2010 | WO | 2009/093838 | A2 | |
| | | | | EP | 2239583 | A2 | |
| | | | | EP | 3869205 | A1 | |
| | | | | CN | 101971035 | A | |
| | | | | KR | 10-2011-0079570 | A | |
| | | | | CN | 103472241 | A | |

Form PCT/ISA/210 (patent family annex) (January 2015)

## REFERENCES CITED IN THE DESCRIPTION

**Patent documents cited in the description**

- JP 2021162325 A